(19) Europäisches Patentamt
European Patent Office
Office européen des brevets

(11) Publication number: **0 250 254 B1**

(12) # EUROPEAN PATENT SPECIFICATION

(45) Date of publication of patent specification: **19.06.91**

(51) Int. Cl.5: **C07C 217/80**, A61K 31/135, C07C 49/223, C07C 47/232

(21) Application number: **87305453.0**

(22) Date of filing: **19.06.87**

(54) **Cis & trans stilbenes and their composition for the treatment of hypertension.**

(30) Priority: **20.06.86 US 876628**

(43) Date of publication of application:
**23.12.87 Bulletin 87/52**

(45) Publication of the grant of the patent:
**19.06.91 Bulletin 91/25**

(84) Designated Contracting States:
**AT BE CH DE ES FR GB GR IT LI LU NL SE**

(56) References cited:
**EP-A- 0 146 271**
**DE-B- 2 047 658**
**GB-A- 1 355 449**

**CHEMICAL ABSTRACTS, vol. 72, no. 5, February 2, 1970, Columbus, Ohio, USA; A.L. MNDZHOYA et al.: "Modification of alkaloid structures. II. Derivatives of o-methylarmepavine, laudanosine, remerine, and laureline with open tetrahydropyridine rings", pages 337, 338, abstract no. 21 808w**

(73) Proprietor: **McNeilab, Inc.**

**Spring House Pennsylvania(US)**

(72) Inventor: **Carson, John Robert**
**551 Rittenhouse, Blvd.**
**Norristown Pennsylvania(US)**

(74) Representative: **Jones, Alan John et al**
**CARPMAELS & RANSFORD 43 Bloomsbury Square**
**London, WC1A 2RA(GB)**

**EP 0 250 254 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention comprises various N-aralkyl derivatives of aminoalkyl stilbenes which are useful as vasodilators and in the treatment of hypertension, e.g. in humans.

Certain phenylethenylbenzylamines are taught in U.S. Patent 3,719,712 and are taught as antiarrhythmic agents. Certain aralkyl(arylethynyl)aralkyl amines are taught in Published European Patent Application No. 146,271 and are taught as vasodilatators and antihypertensives, despite disclosing some acetylene compounds, does not disclose any stilbenes.

### Summary of the Invention

Stilbenes of the following formula (I):

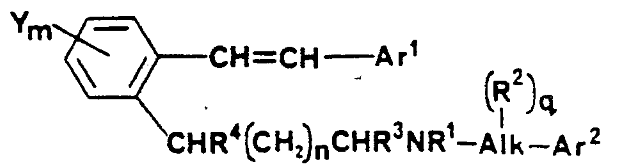

(I)

wherein Y, m, $R^1$, $R^2$, q, Alk, $R^3$, n and $R^4$ are as defined herein and $Ar^1$ and $Ar^2$ contain aromatic moieties have been found to possess vasodilating and anti-hypertensive properties when administered to a mammal in need thereof. Also part of the invention are pharmaceutical compositions containing compounds of the formula (I) and methods of treatment using such compositions.

According to a first aspect of the present invention, there is provided a stilbene derivative of the following formula (I), and the pharmaceutically acceptable acid addition salts and quaternary ammonium compounds thereof, for use in treating angina pectoris or hypertension in a mammal,

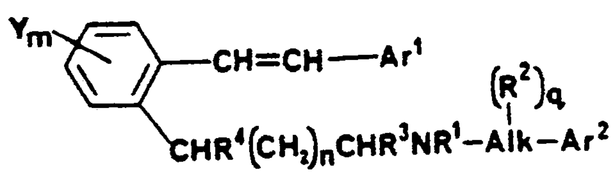

(I)

wherein Y is independently alkyl, alkoxy, alkylthio, alkylsulfinyl, alkylsulfonyl, alkanoyloxy, alkanoylamino, amino, monoalkylamino, dialkylamino, hydroxy, halogen or cyano or methylenedioxy or ethylenedioxy at adjacent ring carbons; m is 0, 1, 2 or 3; $Ar^1$ is phenyl or phenyl independently substituted by one or more of alkyl, alkoxy, alkylthio, alkylsulfinyl, alkylsufonyl, amino, alkylamino, dialkylamino, carboxamido, halogen, fluoroalkyl or cyano; $R^1$ is hydrogen, alkyl, hydroxyalkyl, cycloalkyl, cycloalkylalkyl or -Alk-$Ar^2$; $R^2$ is independently hydroxy, alkyl or phenyl; Alk is straight chain alkylene of 1 to 4 carbons; g is 0, 1 or 2, or 3 if Alk is alkylene of 2 to 4 carbons; $Ar^2$ is a phenyl, phenoxy, thiophenoxy or naphthyl which rings may be substituted independently by one or more of alkyl, alkoxy, alkylthio, hydroxy, halogen, fluoroalkyl, nitro, amino or dialkylamino or by methylenedioxy at adjacent ring carbons; $R^3$ is hydrogen, alkyl or alkoxyalkyl; n is 0, 1 or 2; and $R^4$ is hydrogen or alkyl.

According to a second aspect of the present invention there is provided a pharmaceutical composition comprising a stilbene derivative of formula I as defined above in combination with a pharmaceutically acceptable diluent or carrier.

According to a third aspect of the present invention there is provided a stilbene derivative per se of formula I as defined above provided that: when m = 2, both groups Y are $CH_3O$, $Ar^1$ is p-methoxyphenyl, $R^1$ = methyl, q = 0, n = 0, $R^3$ and $R^4$ are both hydrogen and Alk = methylene; then $Ar^2$ is other than phenyl, p-methoxyphenyl, 3,4-dimethoxyphenyl and 3,4,5-trimethoxyphenyl.

According to a fourth aspect of the present invention there is provided a carbonyl compound of the following formula (IV):

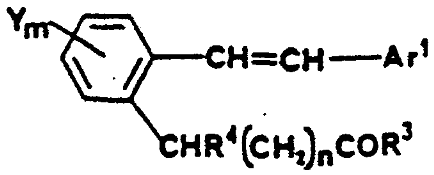

(IV)

wherein Y, m, $Ar^1$, n, $R^3$ and $R^4$ are as defined above.

According to a fifth aspect of the present invention there is provided a process for the preparation of a stilbene derivative of formula I as defined which comprises: a) reducing an acetylene of the following formula (II):

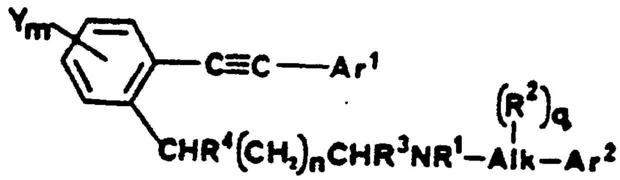

(II)

or

b) reductively alkylating an amine of formula $R^1$-NH-Alk($R^2$)$_q$-$Ar^2$ with a compound of formula (IV) as above; or

c) coupling an amine of the following formula (V):

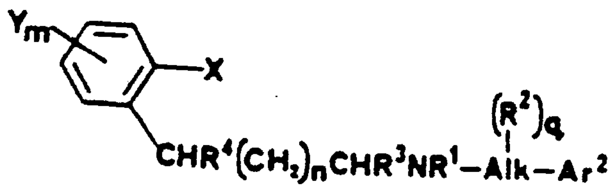

(V)

where X is a halogen with an $Ar^1$-olefin of the formula $Ar^1HC=CH_2$, wherein Y, m, $Ar^1$, $R^1$, $R^2$, $R^3$, $R^4$, n, q, Alk and $Ar^2$ are as defined above.

In particular, Y is alkyl of 1 to 6 carbons such as methyl or ethyl: alkoxy of 1 to 6 carbon atoms such as methoxy or ethoxy: alkylthio of 1 to 6 carbons such as methylthio; alkylsulfinyl of 1 to 6 carbons such as methylsulfinyl: alkylsulfonyl of 1 to 6 carbons such as methylsulfonyl: alkanoyloxy of 2 to 6 carbons such as acetoxy; alkanoylamino of 2 to 6 carbons such as acetylamino; amino; monoalkylamino of 1 to 6 carbons such as ethylamino; dialkylamino of 2 to 12 carbons such as dimethylamino: hydroxy: halogen such as fluoro, chloro or bromo: cyano: or methylenedioxy or ethylenedioxy wherein the two oxygen atoms are attached to two adjacent carbons of the benzene ring. Although the Y groups may be attached at any of the 4 open positions of the benzene ring, particularly preferred are compounds wherein the Y groups are attached at the 4- and/or 5-positions of the ring relative to the amino side chain with the olefin moiety being at the 2-position, 5-methoxy being particularly preferred.

$Ar^1$ is phenyl or a 5- or 6-membered heterocyclic aromatic ring containing 1, 2 or 3, preferably 1 or 2, heteroatoms such as nitrogen, sulphur or oxygen with specific examples being thiophene, pyrrole, furan, pyrazole, imidazole, triazole, oxazole, thiazole, thiodiazole, pyridine, pyridazine, pyrimidine, pyrazine or triazine. Such heterocycles may be attached via a ring carbon atom to the olefinic moiety. The optional substitution on the $Ar^1$ ring is one or more, same or different, of alkyl, alkoxy or alkylthio of 1 to 6 carbons, such as methyl, ethyl, methoxy, iso-propoxy or methylthio; alkylsulfinyl or alkylsulfonyl of 1 to 6 carbons such as methylsulfinyl; amino; alkylamino of 1 to 6 carbons such as methylamino or iso-propylamino; dialkylamino of 2 to 12 carbons, e.g., of 1 to 6 carbons in each alkyl group, such as dimethylamino or N-ethyl-N-n-propylamino; carboxamido of the formula $-CONH_2$; halogen such as fluoro, chloro, or bromo; fluoroalkyl of 1 to 6 carbons and one or more fluoro atoms with examples being 2,2,2-trifluoroethyl and trifluoromethyl: or cyano. Such optional substituents may be attached at any available site on the phenyl or

heterocyclic ring, in particular at the meta and para positions of a phenyl ring relative to the olefin. In particular, $Ar^1$ is unsubstituted phenyl or phenyl substituted by a single substituent such as halo, e.g. para-fluoro.

$R^1$ is hydrogen; alkyl of 1 to 6 carbons such as methyl, ethyl or isopropyl; hydroxyalkyl of 1 to 6 carbons such as 2-hydroxyethyl; cycloalkyl of 3 to 6 carbons such as cyclopropyl or cyclohexyl; or cycloalkylalkyl of 4 to 7 carbons such as cyclopropylmethyl or $R^1$ is independently, i.e., independent of the value for -Alk-$Ar^2$ chosen for these symbols in formula (I), selected from the group consisting of the defined values of -Alk-$Ar^2$, i.e., the entire list of possible -Alk- values and -$Ar^2$ values. For example, $R^1$ can be phenethyl, i.e., $R^1$ is -Alk-$Ar^2$ where Alk is ethylene and $Ar^2$ is phenyl. In particular, $R^1$ is hydrogen or alkyl.

$R^2$ is independently hydroxy; alkyl of 1 to 4 carbons such as methyl, ethyl or iso-propyl; or phenyl.

Alk is methylene, ethylene, trimethylene or tetramethylene.

q is 0, 1, 2 or 3, in particular 0 or 1.

$Ar^2$ is phenyl; phenoxy; thiophenoxy (phenyl-S-); naphthyl, e.g. 1- or 2-naphthyl; or a 5- or 6-membered heterocyclic aromatic ring, preferably one having 1 heteroatom such as nitrogen, sulfur or oxygen, e.g. furan or thiophene attached at the 2 or 3 position, pyrrole attached at the 1, 2 or 3 position and pyridine attached at the 2, 3 or 4 position. The open positions of the ring, or rings in the case of naphthyl, of $Ar^2$ may be substituted by one or more, e.g. one or two, same or different, of alkyl of 1 to 6 carbons such as methyl or ethyl; alkoxy of 1 to 6 carbons such as methoxy and ethoxy; alkylthio of 1 to 6 carbons such as methylthio; hydroxy; halogen such as fluoro, chloro and bromo; fluoroalkyl of 1 to 6 carbons and one or more fluorine atoms with example being 2,2,2-trifluoroethyl and trifluoromethyl; nitro; amino; or dialkylamino of 2 to 12 carbons such as dimethylamino; or methylenedioxy at adjacent ring carbons particularly if $Ar^2$ is phenyl. phenoxy or thiophenoxy, e.g. 3,4-methylenedioxyphenyl. Preferably, $Ar^2$ is phenyl substituted by 1 or 2 substituents such as alkoxy or halo, e.g. 3,4-dimethoxyphenyl or 3,4-dichlorophenyl.

$R^3$ is hydrogen; alkyl of 1 to 6 carbons such as methyl, ethyl, iso-propyl and n-pentyl; or alkoxyalkyl of 1 to 6 carbons in each alkyl portion such as methoxymethyl, n-butoxymethyl and ethoxyethyl.

$R^4$ is in particular, hydrogen; or alkyl of 1 to 6 carbons with examples being methyl, ethyl and n-butyl.

Preferably, at least one of $R^1$ , $R^3$ and $R^4$ is other than hydrogen, e.g. alkyl such as methyl. In particular, at least one of $R^3$ and $R^4$ is other than hydrogen, e.g. methyl. Although not required, Y may be in particular independently alkyl, alkoxy, alkanoyloxy, alkanoylamino, amino, monoalkylamino, dialkylamino, hydroxy, halogen or cyano or methylenedioxy or ethylenedioxy at adjacent ring carbons; $Ar^1$ may be phenyl or a 5- or 6-membered heterocyclic aromatic ring which rings may be independently substituted by one or more of alkyl, alkoxy, amino, alkylamino, dialkylamino, carboxamido, halogen, fluoroalkyl or cyano; and $Ar^2$ is a phenyl, phenoxy, naphthyl or a 5- or 6-membered heterocyclic aromatic ring which rings may be substituted independently by one or more of alkyl, alkoxy, hydroxy, halogen, fluoroalkyl, nitro, amino or dialkylamino or by methylenedioxy at adjacent ring carbons; and m, $R^1$, $R^2$, Alk, q, $R^3$, n and $R^4$ are as described above for formula (I).

The pharmaceutically-acceptable acid-addition salts of the compounds of formula (I) include those of a mineral or organic acid such as hydrochloric, hydrobromic, hydroiodic, sulfuric, phosphoric, fumaric, maleic, cyclohexylsulfamic, citric, lactic, methanesulfonic and similar acids. The term "independently" is used with respect to Y, $Ar^1$ substitution, $R^2$ and $Ar^2$ substitution to indicate that when more than one of such substitution is possible, e.g, when q and m are 2 or 3, such substitution may be different from each other, e.g. when q is 2, one such $R^2$ may be -OH and the other -$CH_3$.

The quaternary ammonium compounds of the compounds of formula (I) include those formed with an alkylhalide or sulfate of about 1 to 6 carbons, e.g. an alkyl bromide or iodide such as methyliodide. The salts and ammonium compounds may be prepared by conventional techniques.

Compounds of formula (I) and other compounds of the invention may exist in various isomeric forms, e.g. in view of the presence of an asymmetric carbon. It is understood that the present invention includes all such individual isomers and their racemates. Also within the scope of the invention are compounds of the invention in the form of hydrates and other solvate forms. Formula (I) compounds exist either in the following cis form of formula (I-Z) or the trans form of formula (I-E):

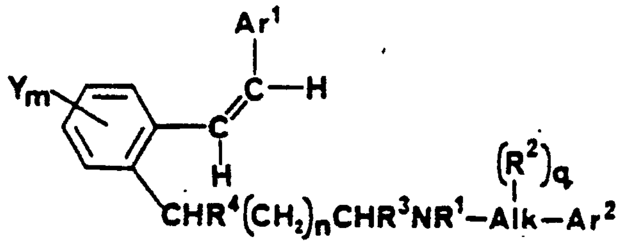

(I-Z)

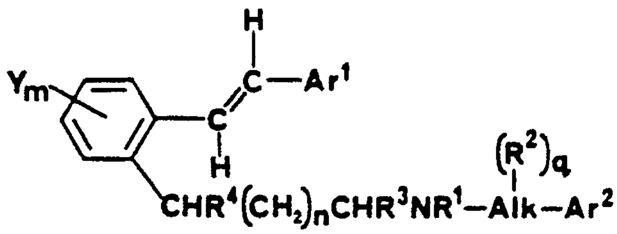

(I-E)

The invention of formula (I) includes either, both and mixtures of such geometrical isomeric forms.

Particular compounds of the invention may be defined as those of formula (I) having one or more of the following definitions: Y is alkoxy, particularly methoxy; m is 0 or 1 and the Y group is at the 5-position of the ring with the aminoalkyl and the olefin moieties at the 1 and 2 positions, respectively; $Ar^1$ is phenyl or phenyl substituted with a single substituent such as chloro; $R^1$ is hydrogen or alkyl such as methyl; $R^2$ is methyl; q is 0 or 1; Alk is methylene or ethylene; $Ar^2$ is phenyl or phenyl with one or two substituents such as alkoxy, e.g. methoxy or chloro; $R^3$ is hydrogen or methyl; n is 0 or 1; and $R^4$ is hydrogen.

Particular compounds of the present invention are the following:

N-[2-(3,4-Dimethoxyphenyl)ethyl]-5-methoxy-α-methyl-2-(2-phenylethenyl)benzenepropanamine, particularly E;

N-[2-(2,6-Dichlorophenyl)ethyl]-5-methoxy-α-methyl-2-(2-phenylethenyl)benzeneethanamine, particularly Z;

N-[2-(3,5-Dimethoxyphenyl)ethyl]-5-methoxy-α-methyl-2-(2-phenylethenyl)benzeneethanamine, particularly Z;

N-[2-(3,4-Dimethoxyphenyl)ethyl]-5-methoxy-α-methyl-2-(2-phenylethenyl)benzeneethanamine, particularly Z;

N-[2-(3,4-Dimethoxyphenyl)ethyl]-5-methoxy-N,α-dimethyl-2-(2-phenylethenyl)benezenepropanamine, particularly E;

N-[2-(3,4-Dimethoxyphenyl)ethyl]-5-methoxy-N,α-dimethyl-2-(2-phenylethenyl)benzenepropanamine, particularly Z;

N-[2-(3,4-Dimethoxyphenyl)ethyl]-5-methoxy-N-methyl-2-(2-phenylethenyl)benzenepropanamine, particularly E;

N-[2-(3,4-Dimethoxyphenyl)ethyl]-5-methoxy-α-methyl-2-(2-phenylethenyl)benzeneethanamine, particularly E;

N-[2-(3,4-Dichlorophenyl)ethyl]-5-methoxy-α-methyl-2-(2-phenylethenyl)benzeneethanamine, particularly E;

N-[2-(3,5-Dimethoxyphenyl)ethyl]-5-methoxy-α-methyl-2-2-(2-phenylethenyl)benzeneethanamine, particularly E;

N-[3-(3,4-Dimethoxyphenyl)propyl]-5-methoxy-α-methyl-(2-phenylethenyl)benzeneethanamine, particularly E; and

N-[2-[2-[2-(4-Fluorophenyl)ethenyl]-5-methoxyphenyl]-1-methylethyl]-1-naphthaleneethanamine, particularly E.

Compounds of formula (I) may be prepared by one of Reaction Schemes (A), (B) or (C) as follows where each of the groups Y, m, $Ar^1$, $R^1$, $R^2$, $R^3$, $R^4$, n, q and $Ar^2$ are as described for formula (I):

Reaction Scheme (A):

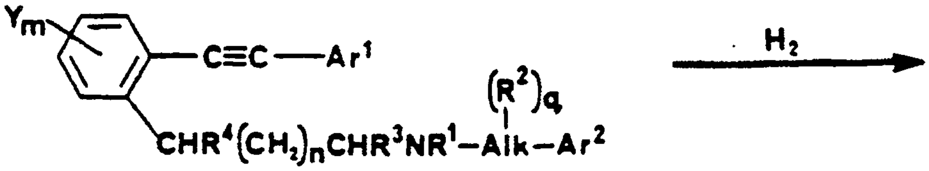


(I)

(II)

Reaction Scheme (B):

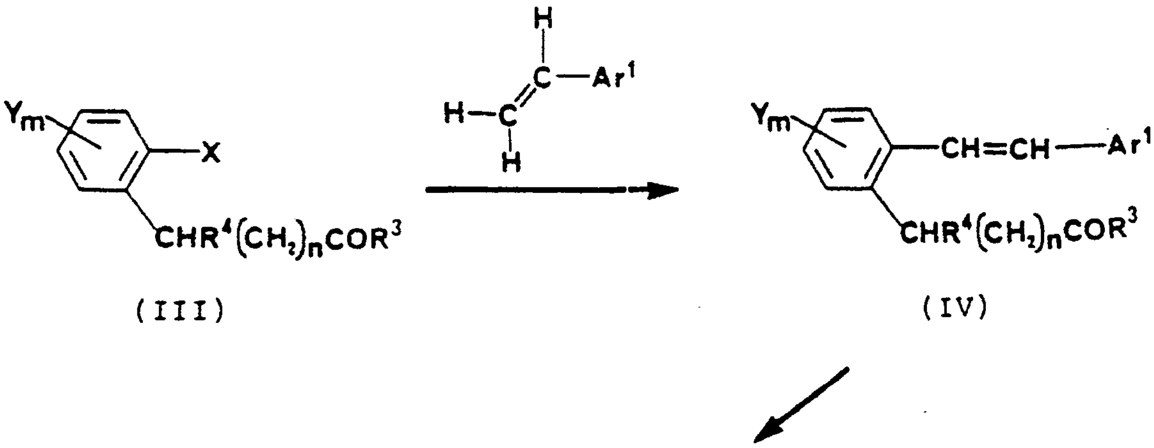

(III)

(IV)

(I)

Reaction Scheme (C):

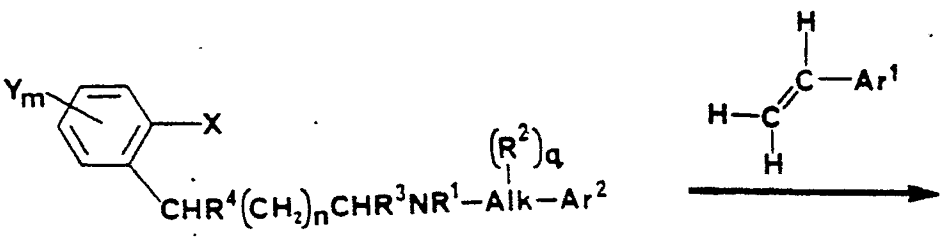

(I)

(V)

In Reaction Scheme (A), an acetylene of the formula (II) is reduced to an olefin by catalytic hydrogenation over a noble metal catalyst such as palladium on $BaSO_4$ known as Lindlar reduction. The reduction may be carried out in the presence of a poison such as pyridine or quinoline. The reduction may be conducted at a hydrogen pressure of about 1.1 to 14.1kgfcm$^{-2}$ (16 to 200 psi) of $H_2$. Starting materials of formula (I) may be prepared as described in European Published Application No. 146,271.

In Reaction Scheme (B), the coupling of the arylhalide (III) where X is halo such as Br or I with an $Ar^1$-$CH=CH_2$ olefin may be accomplished by treating the arylhalide (III) in an ethereal solvent such as THF at -30$^\circ$ C to ambient temperature, as described by R. F. Heck et al. J. Org. Chem., 37, p 2320-2322 (1972). Coupling may also be accomplished by treating the arylhalide (III) with $Ar^1$-$CH=CH_2$ olefin and catalytic quantities, e.g., 0.5 to 10 mole percent, of $Pd[(Ph_3)P]_4$, Pd metal, $Pd(OAc)_2$, $Pd(OAc)_2[P(Ph)_3]_2$ or $PdCl_2[P(Ph)_3]_2$ in an amine solvent such as diethylamine, piperidine, tributylamine, pyrrolidine or triethylamine at ambient temperature. Starting materials of the formula (III) may be prepared as described in

European Published Application No. 146,271.

The second step of Reaction Scheme (B) consists of reductive alkylation of amines of the formula $R^1NH-Alk(R^2)q-Ar^2$ by aldehydes or ketones of the formula (IV) $R^3$ being hydrogen, alkyl or alkoxyalkyl. The reductive alkylation may be carried out in one step from the carbonyl compound and the amine using sodium cyanoborohydride as the reducing agent in a lower alkanol or acetonitrile as the solvent at neutral to mildly acidic pH at temperatures from 0 to 40° C. Reductive alkylation may also be carried out in two steps. The carbonyl compound and amine are first combined and converted to an imine or iminium salt by treatment with molecular sieves or azeotropic removal of water. Reduction is then effected by $NaBH_4$ or $NaCNBH_3$. Using the two step reductive alkylation, the alkyl groups $R^1$ and $Alk(R^2)q-Ar^2$ may be attached sequentially. If $R^1$ is to be methyl, the Eschweiler-Clark procedure using formaldehyde as the carbonyl compound and formic acid or sodium cyanoborohydride as the reducing agent is used.

In Reaction Scheme (C), a halo compound (V) where X is as defined for formula (III) is caused to react with an olefin of the formula $Ar^1HC=CH_2$ under condition similar to those of the first step of Reaction Scheme (B). The formula (V) compound with a fully elaborated amine side chain is charged with appropriately-substituted styrene and an amine such as triethylamine in a pressure bottle together with a noble metal catalyst, such as palladium (II) acetate. The reaction vessel is heated at about 100° C and the product is recovered by filtration and formation of a salt with an acid. Starting materials of the formula (V) for Reaction Scheme (C) can be made as described in European published Application No. 146,271 published June 26, 1985. Regarding the cis/trans distribution of products of Reaction Schemes (A),(B) and (C), Scheme (A) usually results in pure cis, i.e. Z, isomer of the formula (I-Z). Scheme (B) will often result in pure trans, i.e. E,isomer of the formula (I-E) although a mixture will result on occasion. Scheme (C) has been found to produce trans isomer of the formula (I-E). When mixtures are prepared, the individual isomers may be separated by fractional crystallization or chromatography on silica gel. Interconversion of the isomer may be carried out by photoisomerization or by isomerization with iodine in nitrobenzene as described by D. F. DeTar et al. in J. Am. Chem. Soc. 77. 4410 (1955).

The various Y groups in compounds such as those of formulae (I), (II), (III), (IV), and (V), may be transformed among each other by techniques known in the art. For example, when Y is amino, the corresponding compound wherein Y is monoalkylamino may be prepared by acylation with an acyl halide or anhydride to yield the corresponding compound where Y is alkanoylamino followed by hydride reduction with borane or lithium aluminum hydride. When Y is alkylthio, the corresponding compound where Y is alkysulfinyl or alkysulfonyl may be produced by oxidation with hydrogen peroxide or a per acid such as trifluoroperacetic acid by methods known in the art.

Variation in the reaction temperature, reaction time and reactivity of the substrate and particular reagent will all be factors influencing whether the product is the sulfinyl or sulfonyl and manipulation of such variables is well known in the art. When Y is alkoxy, the corresponding compound wherein Y is hydroxy may be produced by conventional dealkylating reagents such as boron tribromide, boron trichloride, trimethylsilyliodide and hydrogen iodide. In addition, compounds wherein Y is alkoxy may be produced from the phenol by alkylation with a reagent such as alkyl halide, e.g., methyl iodide, in the presence of a base.

The above techniques may be modified or substituted by methods generally described in the review article "Synthesis of Stilbenes" by K. B. Becker in Synthesis (5), pages 341-368 (May, 1983). Styrenes of the formula $AR^1HC=CH_2$ may be prepared as described in "Survey of Organic Synthesis, VI" by C. A. Buehler and D. E. Pearson, Wiley Interscience, N.Y. at pages 69-150 (1970).

Compounds of the formula (I), including the acid-addition salts and quaternary compounds thereof, are calcium blockers and as such, are effective against angina and hypertension, particularly as described by S. F. Flaim et al. in "Calcium Blockers - Mechanisms of Action and Clinical Applications", Urban and Schwarzenberg, Baltimore, Md. (1982). Techniques used to determine efficacy as a calcium blocker are described by S. F. Flaim et al. in Pharmacology, Vol. 22, p. 286 to 293 (1981). Compounds of the invention have the advantage of a significant separation between the desirable coronary vasodilator effects and the less desirable side effect of decreased myocardial contractile force.

The activity of compounds of formula (I) for the treatment of hypertension was determined using the Spontaneously Hypertensive Rat (SHR) test as described below.

In this test, the arterial pressure of adult spontaneously hypertensive rats (Charles River) is monitored directly via an aortic cannula. The SH rats are anesthetized with an intravenous anesthetic (methohexital). The left carotid artery is isolated and cannulated. The tip of the cannula is advanced to the aorta and the cannula is exteriorized behind the neck at the level of the scapula. Animals are placed in individual cages and allowed to recover from the anesthetic and are kept unrestrained. The arterial cannula is connected to the pressure transducer which is attached to the recorder. The test compounds are administered to at least 3 rats at doses selected in the range of 0.1 to 100 mg/kg of body weight by intraperitoneal (i.p.) or oral (p.o.)

routes of administration. The arterial pressure and heart rate are monitored for a minimum of 22 hours. A test compound is considered to be active as an antihypertensive agent if the mean arterial pressure (MAP) indicates a fall of >I5 mm of Hg. Each animal serves as its own control.

The results of this test for compounds of formula (I). expressed as "Max Fall BP" (Maximum Fall in Mean Arterial pressure) are shown in Table I.

In addition to their utility in the treatment of hypertension, the compounds of formula (I) are useful in the treatment of the symptoms of angina pectoris by virtue of their ability to dilate coronary arteries. Their activity was measured using the "Langendorff's isolated heart" preparation. This test has been described in "Pharmacological Experiments on Isolated Preparations", Staff of the Department of Pharmacology, University of Edinborough, 2nd Ed., Churchill Livingstone, N.Y., 1970, pp. 112-119. The test compounds were administered at concentrations of 3000, 1000, 300, 100, 30, 10, 3, 1 and 0.3 nanomolar ($10^{-9}$ molar).

The minimum concentration ($EC_{30}$) needed to elicit a 30 percent increase in coronary flow is shown in Table I for compounds of the formula (I) with doses in mg/kg of body weight given p.o. unless otherwise noted.

## Table I

| Product of Example No. | Max Fall BP[a] (dose) | $EC_{30}$ ($\times 10^{-9}$M) |
|---|---|---|
| 2d* | 18 (30) | 55 |
| 3c* | 38 (10)[b] | 57 |
| 4c† | 21 (30) | 2 |
| 5† | 43 (30) | 12 |
| 6e* | 17 (30) | 19 |
| 7e° | 16 (30) | 0.4 |
| 8b† | 77 (30) | 1 |
| | 80 (10) | -- |
| 9† | 90 (30) | 24 |
| 10 | 53 (30)* | 1 (free base) |

* tested as the fumarate salt

† tested as the chloride salt

° tested as the cyclohexylaminosulfonate (hexamate) salt

a) in mm of Hg

b) in mg/kg of body weight given i.p.

For the treatment of hypertension or angina, compounds of the present invention of the formula (I) may be administered orally or parenterally in a pharmaceutical composition comprising about 1 to 2,000 mg, preferably about 30 to 200 mg of one or more of the acetylene compounds per day for an average adult human depending on the activity of the particular compound chosen. The dosage may be divided into I to 4 unit dosage forms per day. While the therapeutic methods of the invention are most useful for human subjects in need of alleviation of hypertension or angina, the compounds may be administered to other mammals at comparable dosages per weight of the subject.

Pharmaceutical compositions containing the olefin compounds of the present invention of formula (I), an acid addition salt thereof or a quaternary ammonium compound thereof as the active ingredient may be prepared by intimately mixing the olefin compound with a pharmaceutical carrier according to conventional

pharmaceutical compounding techniques, which carrier may take a wide variety of forms depending on the form of preparation desired for administration e.g., oral or parenteral. In preparing the compositions in oral dosage form, any of the usual pharmaceutical media may be employed, including liquid carriers such as water, glycols, oils, alcohols and the like for oral liquid preparations such as suspensions, elixers and solutions; and solid carriers such as starches, sugars, kaolin, calcium stearate, ethyl cellulose, etc., including materials which function as lubricants, binders, disintegrating agents and the like for powders, capsules and tablets. Because of their ease in administration, tablets and capsules represent the most advantageous oral dosage form. These compositions employ solid pharmaceutical carriers such as the aforementioned starches, sugars, kaolin and the like, generally with a lubricant such as calcium stearate. It is especially advantageous to formulate the aforementioned pharmaceutical compositions in dosage unit form for ease of administration and uniformity of dosage. The term "dosage unit form" as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets, capsules, pills, powder packets, wafers, teaspoonful, tablespoonful and the like, and segregated multiples thereof.

Also part of the present invention are novel intermediates, e.g., of the formula (IV) and the corresponding E and Z isomers thereof.

In the following Examples, the following abbreviations are used: E (trans); Z (cis); bp (boiling point); mp (melting point); g (grams); ml (milliliters); glc (gas liquid chromatography); hplc (high pressure liquid chromatography); N (normal); M (molar); THF (tetrahydrofuran); MeOH (methanol); DMF (dimethylforamide; 2-PrOH (iso-propanol); EtOAc (ethyl acetate); EtOH (ethanol); $Et_2O$ (diethyl ether); Ph (phenyl); A (angstroms); mmole (millimoles); mg (milligrams); mm (millimeters); hr (hours); min (minutes); psi (pounds per square inch); and C, H, N, etc. (the chemical symbols for the elements). Unless otherwise indicated, all temperatures are reported in degrees centrigrade (°C), all pressures in mm of mercury, all references to $MgSO_4$ and $K_2CO_3$ are to anhydrous materials and all references to ether are to diethyl ether.

Example I

N-[2-(3,4-Dimethoxyphenyl)ethyl]-5-methoxy-α-methyl-2E-(2-phenylethenyl)benzenepropanamine Oxalate

a. 4-(2-Iodo-5-methoxyphenyl)-2-butanone

Samples of iodine (42.4 g, 0.167 mole) and silver acetate (27.87 g, 0.167 mole) were added in portions to a solution of 29.8 g (0.167 mole) of 4-(3-methoxyphenyl)-2-butanone in 167 ml of glacial acetic acid. The mixture was stirred one hour. The silver iodide was removed by filtration and washed with acetic acid. The filtrate was partitioned between ether and water. The ether layer was washed with water, sodium bicarbonate solution and sodium thiosulfate solution. The ether solution was dried with $MgSO_4$ and evaporated to dryness in vacuo. There was obtained 41.8 g (82% yield) of oily 4-(2-iodo-5-methoxyphenyl)-2-butanone.

b. 4-[5-Methoxy-2E-(2-phenylethenyl)phenyl]-2-butanone

A solution of 10.0 g 4-(2-iodo-5-methoxyphenyl)-2-butanone, the product of Example Ia, 5.7 ml of styrene, 1.90 g of $Pd(Ph_3P)_4$ in 4 ml of triethylamine was heated on a steam bath in a pressure bottle over the weekend. The reaction mixture was diluted with MeOH, cooled and filtered. The filtrate was evaporated to a black oil. A flash chromatography eluting with 1:9 acetone:hexane gave 4.3 g of the title compound as a yellow oil in 47% yield.

c. N-[2-(3,4-Dimethoxyphenyl)ethyl]-5-methoxy-α-methyl-2E-(2-phenylethenyl)benzenepropanamine Oxalate

A solution of 4.3 g 4-[5-methoxy-2E-(2-phenylethenyl)phenyl]-2-butanone, the product of Example Ib, 2.8 ml of 3,4-dimethoxybenzeneethanamine and 1.25 g of sodium cyanoborohydride in 40 ml of MeOH was stirred under argon, at room temperature overnight. The solution was brought to pH I with aqueous conc. HCl and evaporated in vacuo. The residue was dissolved in ether, washed with dilute NaOH, water and brine, dried over $K_2CO_3$ and evaporated. The residue was combined with one equivalent of oxalic acid and crystallized from 2-prOH to give the title compound (90% yield) as a white solid, mp = 180-181.5°C.

Example 2

N-[2-(2,6-Dichlorophenyl)ethyl]-5-methoxy-α-methyl-2Z-(2-phenylethenyl)benzeneethanamine (E)-2-Butenedioate (2:l)

a. l-(2-lodo-5-methoxyphenyl)-2-propanone

Using the procedure of Example la and substituting the appropriate ketone for 4-(3-methoxyphenyl)-2-butanone, there was obtained l-(2-iodo-5-methoxyphenyl)-2-propanone, mp = 57-58 ° C.

b. N-[2-(2,6-Dichlorophenyl)ethyl]-2-iodo-5-methoxy-α-methylbenzeneethanamine (E)-2-Butenedioate (l:l)

A solution of 8.0 g (27.6 mmole) of l-(2-iodo-5-methoxyphenyl)-2-propanone, from Example 2a, 5.5 g (29.0 mmole) of 2,6-dichlorobenzeneethanamine and 52 mg (0.28 mmole) of p-toluenesulfonic acid in l00 ml of toluene was heated under reflux with azeotropic removal of water. After four hr, the solvent was evaporated in vacuo. The crude imine was dissolved in l00 ml MeOH and l.4 g (22.l mmole) of sodium cyanoborohydride was added. The mixture was stirred for 20 hr. A methanolic hydrogen chloride solution was added to bring the pH to l. The mixture was stirred for 30 min. The solvent was evaporated in vacuo. The residue was partitioned between ether and 3N NaOH solution. The ether layer was washed with brine, dried and the solvent was evaporated in vacuo. The residue was combined with 3.0 g of fumaric acid in 2-propanol. The precipitated solid was collected to give l0.8 g of the title compound as a white solid, mp = l72-l75 ° C.

c. N-[2-(2.6-Dichlorophenyl)ethyl]-5-methoxy-α-methyl-2-(phenylethynyl)benzeneethanamine (E)-2-Butenedioate (2:l)

To a solution of 8.3 g (l7.2 mmole) of N-[2-(2,6-dichlorophenyl)ethyl]-2-iodo-5-methoxy-α-methyl-benzeneethanamine, the free base of the product of Example 2b, in 80 ml triethylamine under argon was added 2.3 ml (20.7 mmole) of phenylacetylene, 0.40 g (0.34 mmole) of Pd $(Ph_3P)_4$ and 0.l3 g (0.69 mmole) of CuI. The reaction was allowed to stir overnight at room temperature then filtered and evaporated. The residue was washed with 3N NaOH and brine, dried over $K_2CO_3$ and evaporated. The residue was dissolved in methanol and the resulting precipitate filtered. The filtrate was evaporated and the residue dissolved in ether. The resulting precipitate was filtered and the filtrate evaporated to give 8.7 g of crude product free base. The residue was crystallized with 2.2 g of fumaric acid in 95% EtOH. Two more recrystallizations from 95% EtOH gave 2.9 g (33%) of product as a crystalline solid, mp = l87-l88.5 ° C.

d. N-[2-(2,6-Dichlorophenyl)ethyl]-5-methoxy-α-methyl-2Z-(2-phenylethenyl)benzeneethanamine (E)-2-Butenedioate (2:l)

A solution of 2.8 g (6.39 mmole) of N-[2-(2,6-dichlorophenyl)ethyl]-5-methoxy-α-methyl-2-(phenylethynyl)beneethanamine, the free base of the product of Example 2c, in 50 ml of pyridine was hydrogenated at atmospheric pressure over 0.ll g of 5% Pd/$BaSO_4$ until one equivalent of hydrogen was taken up. The mixture filtered through Dicalite and the filtrate evaporated. The residue was combined with one equivalent (0.68 g) of fumaric acid and crystallized from l:l 95% EtOH:2-PrOH. One recrystallization from 95% EtOH gave l.88 g of the title compound (59% yield), mpl60-l63 ° C.

Example 3

(N-[2-(3,5-Dimethoxyphenyl)ethyl]-5-methoxy-,-methyl-2Z-(2-phenylethenyl)benzeneethanamine

a. l-[5-methoxy-2-(phenylethnyl)phenyl]-2-propanone

A solution of 5.5 g (l8.9 mmole) l-(2-iodo-5-methoxy-phenyl)-2-propanone, the product of Example 2a, 4.l5 ml (37.8 mmole) of phenylacetylene, 0.037 g of cuprous iodide (l mole percent) and 0.066 g of bis-(triphenylphosphine) palladium (II) chloride (5 mole percent) in 60 ml of triethylamine was stirred for l8 hr under $N_2$ at room temperature. The mixture was partitioned between water and ether. The ether layer was washed successively with cold hydrochloric acid solution, water and brine. It was dried over $MgSO_4$ and the solvent evaporated in vacuo to give 5.6 g (l00% yield) of brown crystals.

Recrystallization from methylcyclohexane afforded pure l-[5-methoxy-2-(phenylethynyl)phenyl]-2-propanone, mp = 60-62 ° C.

b. N-[2-(3,5-Dimethoxyphenyl)ethyl]-5-methoxy-α-methyl-2-(phenylethynyl)benzeneethanamine (E)-2-Butenedioate (2:l)

To a 60 g sample of 5A molecular sieves in l00 ml of toluene were added 8.5 g (32 mmole) of l-[5-methoxy-2-(phenylethynyl)phenyl]-2-propanone from Example 3a and 5.82 g (32 mmole) of 3,5-dimethoxybenzeneethanamine were added. The mixture was stirred for two days at room temperature. The sieves were filtered and washed with toluene. The solvent was evaporated in vacuo. The residue was taken up in 50 ml of MeOH and 0.4 g (l0.6 mmole) of sodium borohydride (pellet) was added. After 90 min water was added. The mixture was partitioned between $CH_2Cl_2$ and water. The organic solution was washed with diluted hydrochloric acid, NaOH solution and water. The solution was dried ($K_2CO_3$) and the solvent evaporated in vacuo. The residue was taken up in MeOH and 2.76 g of fumaric acid added. The solid (9.l4 g. 63% yield) was collected and dried to give the title compound as a white solid, mp = l85-l90° C (decomposition).

c. N-[2-(3,5-Dimethoxyphenyl)ethyl]-5-methoxy-α-methyl-2Z-(2-phenylethenyl)benzeneethanamine (E)-2-Butenedioate (2:l)

Using the procedure of Example 2d and employing an equivalent quantity of N-[2-(3,5-dimethoxyphenyl)ethyl]-5-methoxy-α-methyl-2-(phenylethynyl)benezeneethanamine, the free base of Example 3b. in place of N-[2-(2,6-dichlorophenyl)ethyl]-5-methoxy-α-methyl-2-(phenylethynyl)benzeneethanamine, there was obtained a 77% yield of the title compound, mp = l73-l75° C.

Example 4

N-[2-(3,4-Dimethoxyphenyl)ethyl]-5-methoxy-α-methyl-2Z-α-(2-phenylethenyl)-beneethanamine

a. N-[2-(3,4-Dimethoxyphenyl)ethyl]-2-iodo-5-methoxy-α-methylbenzeneethanamine Hydrochloride

A mixture of 20.0 g (0.06 mole) of l-(2-iodo-5-methoxy-phenyl)-2-propanone, the product of Example 2a, and ll.9 ml (0.07 mole) of 3,4-dimethoxybenzeneethanamine in 200 ml of chloroform was stirred overnight over 240 g of 5A molecular sieves. After filtering, the solvent was evaporated in vacuo to give 26.3 g of the corresponding imine. The imine was dissolved in 200 ml of MeOH and 4 ml of methanolic hydrogen chloride and 2.9l g (0.046 mole) of sodium cyanoborohydride were added. The mixture was stirred for 3 days under an atmosphere of $N_2$. Additional methanolic hydrogen chloride was added over a several hour period to pH l. The mixture was allowed to stand at room temperature overnight and the solvent evaporated in vacuo. The residue was partitioned between ether and aqueous sodium hydroxide. The ether layer was washed with water and brine, dried ($K_2CO_3$) and evaporated in vacuo. The residue was treated with ethereal hydrogen chloride to give the hydrochloride salt. It was recrystallized successively from 2-propanol and ethanol to afford N-[2-(3,4-dimethoxyphenyl)ethyl]-2-iodo-5-methoxy-α-methylbenzeneethanamine hydrochloride, mp = l70-l72° C.

b. N-[2-(3,4-Dimethoxyphenyl)ethyl]-5-methoxy-α-methyl-2-(phenylethynyl)benzeneethanamine (E)-2-Butenedioate Hydrate (6:3:2)

To a solution of 7.0 ml (63.3 mmole) of phenylacetylene in l20 ml THF was added l.6 m n-butyl lithium in hexane until the solution turned green. The solution was stirred l5 min then added by cannula to a solution of 8.6 g (63.3 mmole) zinc chloride in 240 ml of THF. After ten min, 24.0 g (52.7 mmole) of N-[2-(3,4-dimethoxyphenyl)ethyl]-2-iodo-5-methoxy-α-methylbenzeneethanamine, the free base of the product of Example 4a, in l20 ml of THF was added, followed by l.0 g of $Pd(Ph_3P)_4$ and the solution allowed to stir overnight. The reaction was filtered and the filtrate evaporated. The residue was dissolved in MeOH and the resulting precipitate filtered. The filtrate was evaporated and the residue dissolved in $CH_2Cl_2$ and washed with $H_2O$ and brine and dried over $K_2CO_3$. The $CH_2Cl_2$ was evaporated to give 24.6 g of residue which was combined with 5.8 g of fumaric acid in 95% EtOH to give l2.8 g (45%) of product as a crystalline solid, mp = l65-l67° C.

c. N-[2-(3,4-Dimethoxyphenyl)ethyl]-5-methoxy-α-methyl-2Z-(2-phenylethenyl)benzeneethanamine Hydrochloride

Using the procedure of Example 2d and employing an equivalent quantity of N-[2-(3,4-dimethoxyphenyl)ethyl]-5-methoxy-α-methyl-2-(phenylethynyl)benzeneethanamine, the free base from Example 4b, in place of N-[2-(2,6-dichlorophenyl)ethyl]-5-methoxy-α-methyl-2-(phenylethynyl)benezeneethanamine and 3.4 times as much 5% Pd/$BaSO_4$ on a w/w basis. There was obtained a 77% yield of the title compound, mp=142.5-145 °C.

Example 5

N-[2-(3,4-Dimethoxyphenyl)ethyl]-5-methoxy-N,α-dimethyl-2E-(2-phenylethenyl)benezenepropanamine Hydrochloride

A solution of 6.1 g of N-[2-(3,4-dimethoxyphenyl)ethyl]-5-methoxy-α-methyl-2E-(2-phenylethenyl)-benezenepropanamine free base from Example 1c, 4.1 ml of 37% formaldehyde and 1.04 g sodium cyanoborohydride in 60 ml of MeOH was stirred at room temperature. After two hours, 12 ml of glacial acetic acid was added. The solution was stirred for one hour and evaporated. The residue was dissolved in $CH_2Cl_2$ washed with dilute NaOH and brine, dried with $K_2CO_3$ and evaporated. The residue was dissolved in ether and ethereal HCl added. The solution was cooled, the solvent decanted and the residue crystallized from EtOAc. Three recrystallizations from 95% EtOH/$Et_2O$ gave the title compound (48% yield), mp=156-159 °C.

Example 6

N-[2-(3,4-Dimethoxyphenyl)ethyl]-5-methoxy-N,α-dimethyl-2Z-(2-phenylethenyl)benzenepropanamine (E)-2-Butenedioate (2:1)

a. 2-lodo-5-methoxy-N,α-dimethylbenzenepropanamine Hydrochloride

A solution of 19.41 g (0.625 mole) of methylamine in 400 ml of anhydrous methanol was treated with 37.92 g (0.125 mole) of 4-(2-iodo-5-methoxyphenyl)-2-butanone, the product of Example 1a, 6.28 g (0.1 mole) of sodium cyanoborohydride and 21.07 g (0.312 mole) of methylamine hydrochloride and allowed to stir at room temperature under an atmosphere of nitrogen for 2-1/2 hours. The resulting mixture was cooled in an ice bath and treated with concentrated hydrochloric acid to pH 1. The solvent was evaporated in vacuo and the residue partitioned between 1.5 l of water and 700 ml of ether. The water layer was separated and treated with 50% sodium hydroxide solution until basic. The resulting crude product was extracted into 500 ml of ether, washed with brine, and the brine layer extracted with an additional 400 ml portion of ether. The combined ether layers were dried over anhydrous potassium carbonate and evaporated in vacuo to yield 31.09 g (78%) of crude oil. The oil was dissolved in methanol and treated with ethereal hydrogen chloride. Ether was added to the mixture and the resulting hydrochloride salt separated by filtration to give 19.75 g (44%) of crude product. One recrystallization from 2-propanol yielded pure 2-iodo-5-methoxy-N-α-dimethylbenzenepropanamine hydrochloride, a white solid, mp=189-190 °C.

b. N-[3-(2-Iodo-5-methoxyphenyl)-1-methylpropyl]-3,4-dimethoxy-N-methylbenzeneacetamide

A solution of 17.87 g (0.056 mole) of 2-iodo-5-methoxyN,α-dimethylbenzenepropanamine. the free base of Example 6a, in 58 ml of dry methylene chloride was added dropwise to a cooled (5 °C) solution of 3,4-dimethoxyphenylacetyl chloride in 68 ml of dry methylene chloride over a period of five minutes while stirring under an atmosphere of nitrogen. After about two min of stirring, 5.67 g (0.056 mole) of triethylamine was added followed by an additional 20 ml of dry methylene chloride. The reaction mixture was allowed to warm to room temperature while stirring overnight. An additional 80 ml of methylene chloride was added and the mixture washed successively with 200 ml of water, 300 ml of 5% hydrochloric acid, and 200 ml of sodium hydroxide solution. The organic phase was dried over anhydrous magnesium sulfate and evaporated in vacuo to yield 27.5 g (99%) of N-[3-(2-iodo-5-methoxy-phenyl)-1-methylpropyl]-3,4-dimethoxy-N-methylbenzene-acetamide as an orange gum.

c. N-[2-(3,4-Dimethoxyphenyl)ethyl]-2-iodo-5-methoxy-N,α-dimethylbenzenepropanamine

A solution of 27.19 g (0.055 mole) of N-[3-(2-iodo-5-methoxyphenyl)-1-methylpropyl]-3,4-dimethoxy-N-methyl-benzeneacetamide, the product of Example 6b, was added dropwise over a 30 min period with stirring to 165 ml of 1M $BH_3$•THF under an atmosphere of nitrogen. The mixture was heated to reflux for 2.5

hr and then cooled to 0° C in an ice bath. A mixture of 50 ml THF and 25 ml of water was added carefully and the resulting mixture was evaporated in vacuo to remove the THF. The residue was treated with 100 ml of water and 5.79 g of sodium hydroxide and heated to reflux for four hr. Heating was interrupted and an additional 10 g of sodium hydroxide was added. Heating was resumed for another hr. The mixture was cooled and extracted with two 300 ml portions of ether. The combined ether extracts were washed with water, brine, and dried over anhydrous potassium carbonate. The solvent was removed in vacuo to give 27.66 g of a yellow oil. The oil was partitioned between ether and 400 ml of 10% hydrochloric acid, at which time the resulting oil was separated, dissolved in a large volume of water, and washed with ether. The aqueous phase was treated with 10% sodium hydroxide solution until basic and extracted several times with ether. The combined ether layers were dried over anhydrous potassium carbonate and evaporated in vacuo to yield 20.98 g (79%) of a yellow oil.

The oil was converted to a crystalline oxalate salt from methanol, mp = 189-190° C. The oxalate salt was partitioned between methylene chloride and water and treated with a slight excess of sodium hydroxide. The methylene chloride layer was separated, dried over anhydrous potassium carbonate, and evaporated in vacuo to yield 16.33 g of N-[2-(3,4-dimethoxyphenyl)ethyl]-2-iodo-5-methoxy-N,α-dimethylbenzenepropanamine as a colorless oil.

d. N-[2-(3,4-Dimethoxyphenyl)ethyl]-5-methoxy-N,α-dimethyl-2-(phenylethynyl)benzenepropanamine Hydrochloride.

A solution of 4.55 ml (41.4 mmole) of phenylacetylene in 40 ml of dry THF is cooled to 0° C and 21.4 ml of 1.9 M n-butyllithium in hexane (41.4 mmole) is added. The resulting solution is stirred for 15 min under $N_2$ then transferred to a flask containing 5.53 g (40.6 mmole) of dry $ZnCl_2$. The mixture is stirred 15 min at 0° C and a solution of 33.8 mmole of N-[2-(3,4-dimethoxyphenyl)ethyl]-2-iodo-5-methoxy-N,α-dimethylbenzenepropanamine from Example 6c and 0.78 g (0.68 mmoles) of tetrakis(triphenylphosphine) palladium (0) in 100 ml of THF is added. The mixture was allowed to warm to room temperature and stirred for 24 hr. The mixture is diluted with $CH_2Cl_2$ and washed with water, 5% HCl, dilute NaOH, dried ($K_2CO_3$) and the solvent evaporated in vacuo. The residue is dissolved in MeOH and ethereal hydrogen chloride added. The solvent was evaporated in vacuo and the residue crystallized from 2-PrOH/ether. The solid was recrystallized from 2-PrOH/ether to give 5.53 g (33% yield) of N-[2-(3,4-dimethoxyphenyl)ethyl]-5-methoxy-N,α-dimethyl-2-(phenylethynyl)benzenepropanamine hydrochloride, mp = 119-122° C.

e. N-[2-(3,4-Dimethoxyphenyl)ethyl]-5-methoxy-N,α-dimethyl-2Z-(2-phenylethenyl)benzenepropanamine (E)-2-Butenedioate (2:1)

A Parr bottle containing 0.44 g (4% w/w) of 5% $Pd/BaSO_4$ and 10.9 g (23.8 mmole) of N-[2-(3,4-dimethoxyphenyl)ethyl]-5-methoxy-N,α-dimethyl-2-(phenylethynyl)benzenepropanamine, from Example 6d, in 200 ml of pyridine was shaken on the Parr hydrogenator, starting at 42 psi, until all the starting material had disappeared. The reaction mixture was filtered thru Dicalite and the filtrate evaporated to yield a light oil. The oil was combined with one equivalent of fumaric acid and crystallized from 2-PrOH. One recrystallization from 95% EtOH gave 8.7 g of the title compound (71% yield), mp = 136-138° C.

Example 7

N-[2-(3,4-Dimethoxyphenyl)ethyl]-5-methoxy-N-methyl-2E-(2-phenylethenyl)benzenepropanamine Biscyclohexylsulfamate

a. 2-Iodo-5-methoxybenzenepropanoic Acid

Samples of iodine (192.6 g, 0.759 mole) and silver acetate (126.7 g, 0.759 mole) were added in portions over 20 min to a solution of 138.6 g (0.759 mole) of 3-methoxy-benezenepropanoic acid in 750 ml glacial acetic acid. An additional 250 ml of glacial acetic acid was added. The mixture became warm and was stirred for one hour. The precipitated silver iodide was filtered and washed with acetic acid and the filtrate was poured into ice water and the solid collected. The solid was taken up in ether, washed with sodium thiosulfate solution and brine, dried with $MgSO_4$ and the solvent evaporated in vacuo. The residue was recrystallized from $CHCl_3$/ligroin to give 148.7 (64% yield) of 2-iodo-5-methoxybenzenepropanoic acid, mp = 105-106° C.

b. 2-Iodo-5-methoxybenzenepropanoyl chloride

To a solution of l3.0 g (0.042 mole) of 2-iodo-5-methoxybenzenepropanoic acid, the product of Example 7a, and 4 ml of DMF in 80 ml of dry toluene at 0° C was added 4.0 ml (0.046 mole) of oxalyl chloride over l5 min. The reaction was stirred overnight to give a solution of 2-iodo-5-methoxybenzenepropanoyl chloride in toluene.

c. N-[2-(3,4-Dimethoxyphenyl)ethyl]-2-iodo-5-methoxy-N-methylbenzenepropanamide

A solution of l3.8 g (0.042 mole) of 2-iodo-5-methoxybenzenepropanoyl chloride, the product of Example 7b, in 80 ml of toluene was cooled to 0° C and 24.6 g (0.l26 mole) of N-methylhomoveratrylamine was added over a l5 min period. An additional 50 ml of toluene was added. The temperature was allowed to warm to room temperature and stirring continued for 3-l/2 hr. The mixture was partitioned between 500 ml of methylene chloride and 400 ml of water. The methylene chloride layer was separated and washed with 400 ml of 5% hydrochloric acid followed by a washing with 400 ml of 5% sodium hydroxide solution. The organic phase was dried over anhydrous magnesium sulfate and evaporated in vacuo to yield N-[2-(3,4-dimethoxyphenyl)ethyl]-2-iodo-5-methoxy-N-methylbenzenepropanamide, a pale yellow oil that partially crystallized on standing.

d. N-[2-(3,4-Dimethoxyphenyl)ethyl]-2-iodo-5-methoxy-N-methylbenzenepropanamine 4-Methylbenzene Sulfonate

A slurry of N-[2-(3,4-dimethoxyphenyl)ethyl]-2-iodo5-methoxy-N-methylbenzenepropanamide, the product of Example 7c, in l80 ml of THF was added over a l5 min period to l26 ml (0.l26 mole) of l M $BH_3$•THF solution under $N_2$. The mixture was heated to reflux for two hours. The reaction mixture was cooled in an ice/water bath and quenched by addition of a mixture of 25 ml of water and 25 ml THF. The solvents were evaporated in vacuo, the residue treated with 50 ml of propionic acid and heated to reflux for l-l/2 hr. After cooling to room temperature, the mixture was partitioned between 600 ml of water and 400 ml of ether. The pH was adjusted to pH 8 by addition of 50% sodium hydroxide solution. The aqueous phase was separated and further basified with sodium hydroxide to pH l2 followed by extraction with 200 ml of ether. The combined ether extracts were washed with two 500 ml portions of l0% sodium hydroxide solution and dried over anhydrous potassium carbonate. The solvent was removed in vacuo to yield a colorless oil. The oil was partially dissolved in methanol and the insoluble materials present removed by filtration through diatomaceous earth. The resulting clear solution was treated with p-toluenesulfonic acid until neutral and the solvent removed in vacuo. The resulting residue was treated with ethanol and evaporated in vacuo two times. The residue was then dissolved in 40 ml of hot EtOH and filtered through diatomaceous earth. The filtrate was treated with ether to the cloud point and the mixture allowed to crystallize at room temperature. The resulting crystals were filtered and washed with ether to yield l0.6 g (39%) of crude product. One recrystallization from EtOH/$Et_2O$ afforded pure N-[2-(3,4-dimethoxyphenyl)-ethyl]-2-iodo-5-methoxy-N-methylbenzenepropanamine 4-methylbenzenesulfonate, a white solid, mp = l05-l06.5° C.

e. N-[2-(3,4-Dimethoxyphenyl)ethyl]-5-methoxy-N-methyl-2E-(2-phenylethenyl)benzenepropanamine Biscyclohexylsulfamate

A solution of 2.43 g N-[2-(3,4-dimethoxyphenyl)ethyl]-2-iodo-5-methoxy-N-methylbenzenepropanamine, the free base of the product of Example 7d, and 0.9 ml styrene in triethylamine in a pressure bottle was deoxygenated with nitrogen and 0.06 g palladium (II) acetate added. The solution was heated on a steam bath for five days. The reaction mixture was diluted with $Et_2O$, filtered, washed with dilute NaOH, water and brine, dried with $K_2CO_3$ and evaporated to an orange-brown oil. An hplc chromatography eluting with 2:l:7 $CH_2Cl_2$:MeOH:n-$C_6H_{14}$ gave a light orange oil which was combined with two equivalents of cyclohexylsulfamic acid and crystallized from 2-PrOH. One recrystallization from 2-PrOH gave the title compound (38% yield), mp = l08-ll0° C.

Example 8

N-[2-(3,4-Dimethoxyphenyl)ethyl]-5-methoxy-α-methyl-2E-(2-phenylethenyl)benzeneethanamine Hydrochloride

a. [5-Methoxy-2E-(2-phenylethenyl)phenyl]-2-propanone

Using the procedure of Example lb and employing an equivalent quantity of l-(2-iodo-5-methoxyphenyl)-2-propanone, the product of Example 2a, in place of 4-(2-iodo-5-methoxyphenyl)-2-butanone, there was obtained a 54% yield of the title compounds as a yellow oil.

b. N-[2-(3,4-Dimethoxyphenyl)ethyl]-5-methoxy-α-methyl-2E-(2-phenylethenyl)benzeneethanamine Hydrochloride

Using the procedure of Example lc and employing an equivalent quantity of [5-methoxy-2E-(2-phenylethenyl)-phenyl]-2-propanone, the product of Example 8a, in place of 4-[5-methoxy-2E-(2-phenylethenyl)phenyl]-2-butanone, there was obtained a 40% yield of the title compound, m.p. = l70-l72 ° C.

Example 9

N-[2-(3,4-Dichlorophenyl)ethyl)-5-methoxy-α-methyl-2E-(2-phenylethenyl)benzeneethanamine Hydrochloride

Using the procedure of Example lc and employing an equivalent quantity of 3,4-dichlorobenzeneethanamine in place of 3,4-dimethoxybenzeneethanamine and using the stilbene product of Example 8a in the place of the product of Example lb, there was obtained a 30% yield of the title compound, mp = 220-222 ° C.

Example l0

N-[2-(3,5-Dimethoxyphenyl)ethyl]-5-methoxy-α-methyl-2E-(2-phenylethenyl)benzeneethanamine (E)-2-Butendioate (3:5)

Using the procedure of Example 9 and employing an equivalent quantity of 3,5-dimethoxybenzeneethanamine in place of 3,4-dichlorobenzeneethanamine, there was obtained a 48% yield of the title compound, mp = l72-l73 ° C.

Example ll

N-[3-(3,4-Dimethoxyphenyl)propyl,-5-methoxy-α-methyl-2E-(2-phenylethenyl)benzeneethanamine Hydrochloride

Using the procedure of Example 9 and employing an equivalent quantity of 3,4-dimethoxybenzenepropanamine in place of 3,4-dichlorobenzeneethanamine, there was obtained a 3l% yield of the title compound, mp = l55-l57 ° C.

Example l2

N-[2-[2E-(4-Fluorophenyl)ethenyl]-5-methoxyphenyl]-l-methylethyl]-l-naphthaleneethanamine (E)-2-Butenedioate (2:l)

a. l-[2E-[2-(4-Fluorophenyl)ethenyl]-5-methoxyphenyl]-2-propanone

A solution of l0.0 g of l-(2-iodo-5-methoxyphenyl)-2-propanone, the product of Example 2a, and 6.3 ml of 4-fluorostyrene in 5 ml of triethylamine in a pressure bottle was degassed with $N_2$ and 0.08 g of palladium (II) acetate added. The solution was heated on a steam bath for 3 days. The reaction mixture was diluted with ether, washed with dilute NaOH, water and brine, dried with $MgSO_4$ and evaporated to yield an orange-brown oil. An hplc chromatograph eluting with l:7 acetone:hexane gave a yellow and white crystalline material which was recrystallized with ether/hexane to give the title compound (20% yield) as a white solid, mp = 64-66 ° C.

b. N-[2-[2E-[2-(4-Fluorophenyl)ethenyl]-5-methoxyphenyl]-l-methylethyl,-l-naphthalene-ethanamine (E)-2-Butenedioate (2:l)

A solution of l.96 g of [2E-[2-(4-fluorophenyl)ethenyl]-5-methoxyphenyl]-2-propanone, the product of Example l2a, l.l8 g of l-naphthaleneethanamine and 0.6 g of sodium cyanoborohydride in 20 ml of MeOH was stirred at room temperature overnight. The reaction was filtered and evaporated in vacuo. The residue was dissolved in $CH_2Cl_2$, washed with dilute NaOH and water, dried with $K_2CO_3$ evaporated to a yellow oil. The oil was combined with one equivalent of fumaric acid and crystallized from MeOH. One recrystallization from 95% EtOH gave the title compound (43% yield), mp = l95-20l° C.

## Claims

1. A stilbene derivative of the following formula (I), and the pharmaceutically acceptable acid addition salts and quaternary ammonium compounds thereof, for use in treating angina pectoris or hypertension in a mammal,

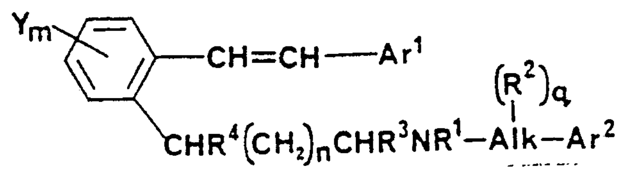

(I)

wherein

- Y is independently alkyl, alkoxy, alkylthio, alkylsulfinyl, alkylsulfonyl, alkanoyloxy, alkanoylamino, amino, monoalkylamino, dialkylamino, hydroxy, halogen or cyano or methylenedioxy or ethylenedioxy at adjacent ring carbons;
- m is 0, 1, 2 or 3;
- $Ar^1$ is phenyl or phenyl independently substituted by one or more of alkyl, alkoxy, alkylthio, alkylsulfinyl, alkylsufonyl, amino, alkylamino, dialkylamino, carboxamido, halogen, fluoroalkyl or cyano;
- $R^1$ is hydrogen, alkyl, hydroxyalkyl, cycloalkyl, cycloalkylalkyl or -Alk-$Ar^2$;
- $R^2$ is independently hydroxy, alkyl or phenyl;
- Alk is straight chain alkylene of 1 to 4 carbons;
- q is 0, 1 or 2, or 3 if Alk is alkylene of 2 to 4 carbons;
- $Ar^2$ is a phenyl, phenoxy, thiophenoxy or naphthyl which rings may be substituted independently by one or more of alkyl, alkoxy, alkylthio, hydroxy, halogen, fluoroalkyl, nitro, amino or dialkylamino or by methylenedioxy at adjacent ring carbons;
- $R^3$ is hydrogen, alkyl or alkoxyalkyl;
- n is 0, 1 or 2; and
- $R^4$ is hydrogen or alkyl.

2. The stilbene derivative of Claim 1, for use in treating angina pectoris or hypertension in a mammal, wherein

- y is alkyl of 1 to 6 carbons; alkoxy of 1 to 6 carbons; alkylthio of 1 to 6 carbons; alkylsufinyl of 1 to 6 carbons; alkylsulfonyl of 1 to 6 carbons; alkanoyloxy of 2 to 6 carbons; alkanoylamino of 1 to 6 carbons; amino; monoalkylamino of 1 to 6 carbons; dialkylamino of 2 to 12 carbons; hydroxy; fluoro, chloro or bromo; cyano; or methylenedioxy or ethylenedioxy;
- m is 0, 1, 2, or 3;
- $Ar^1$ is phenyl or phenyl substituted with one or more of alkyl, alkoxy, alkylthio, alkylsulfinyl, alkylsufonyl or alkylamino of 1 to 6 carbons each, amino, dialkylamino of 2 to 12 carbons, carboxamido, fluoro, chloro or bromo, fluoroalkyl of 1 to 6 carbons or cyano;
- $R^1$ is hydrogen, alkyl of 1 to 6 carbons, hydroxyalkyl of 1 to 6 carbons, cycloalkyl of 3 to 6 carbons, cycloalkylalkyl of 4 to 7 carbons or -Alk-$Ar^2$;
- $R^2$ is hydroxy, alkyl of 1 to 4 carbons or phenyl;
- q is 0 or 1;
- Alk is methylene, ethylene, trimethylene or tetramethylene;
- $Ar^2$ is phenyl, phenoxy, thiophenoxy or naphthyl which rings may be substituted by one or more of alkyl, alkoxy, or alkylthio of 1 to 6 carbons each, hydroxy, fluoro, chloro, bromo, fluoroalkyl of 1 to 6 carbons, nitro, amino, dialkylamino of 2 to 12 carbons or methylenedioxy;

$R^3$ is hydrogen, alkyl of 1 to 6 carbons or alkoxyalkyl of 1 to 6 carbons in each alkyl portion;
n is 0, 1 or 2; and
$R^4$ is hydrogen or alkyl of 1 to 6 carbons.

**3.** The stilbene derivative of claim 1 or claim 2, for use in treating angina pectoris or hypertension in a mammal, which is a cis stilbene of the following formula (I-Z):

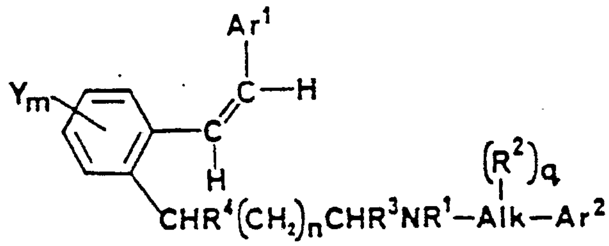

(I-Z)

**4.** The stilbene derivative of claim 1 or claim 2, for use in treating angina pectoris or hypertension in a mammal, which is a trans stilbene of the following formula (I-E):

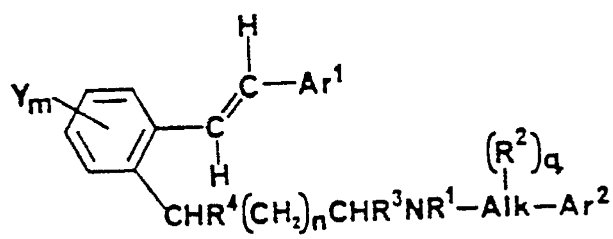

(I-E)

**5.** The stilbene derivative of claim 1, for use in treating angina pectoris or hypertension in a mammal, which is:
N-[2-(3,4-dimethoxyphenyl)ethyl]-5-methoxy-α-methyl-2-(2-phenylethenyl) benzenepropanamine
N-[2-(2, 6-dichlorophenyl)ethyl]-5-methoxy-α-methyl-2-(2-phenylethenyl) benzeneethanamine;
N-[2-(3,5-dimethoxyphenyl)ethyl]-5-methoxy-α-methyl-2-(2-phenyletheneyl) benzeneethanamine;
N-[2-(3,4-dimethoxyphenyl) ethyl]-5-methoxy-α-methyl-2-(2-phenylethenyl) benzeneethanamine;
N-[2-(3, 4-dimethoxyphenyl)ethyl]-5-methoxy-N, α-dimethyl-2-(2-phenylethenyl) benezenepropanamine;
N-[2-(3, 4-dimethoxyphenyl) ethyl]-5-methoxy-N, α-dimethyl-2-(2-phenylethenyl) benzenepropanamine;
N-[2-(3,4-dimethoxyphenyl)ethyl]-5-methoxy-N-methyl-2-(2-phenylethenyl) benzenepropanamine;
N-[2-(3, 4-dimethoxyphenyl) ethyl]-5-methoxy-α-methyl-2-(2-phenylethenyl) benzeneethanamine;
N-[2-(3, 4-dichlorophenyl)ethyl]-5-methoxy-α-methyl-2-(2-phenylethenyl) benzeneethanamine;
N-[2-(3,5-dimethoxyphenyl)ethyl]-5-methoxy-α-methyl-2-(2-phenylethenyl) benzeneethanamine;
N-[3-(3,4-dimethoxyphenyl)propyl]-5-methoxy-α-methyl-2-(2-phenylethenyl) benzeneethanamine;
N-[2-[2-[2-(4-fluorophenyl)ethenyl]-5-methoxyphenyl]-1-methylethyl]-l-naphthaleneethanamine;
N-[2-(3 , 4-dimethoxyphenyl)ethyl]-5-methoxy-α-methyl-2E-(2-phenylethenyl) benzenepropanamine;
N-[2-(2, 6-dichlorophenyl) ethyl]-5-methoxy-α-methyl-2Z-(2-phenylethenyl) benzeneethanamine;
N-[2-(3, 5-dimethoxyphenyl)ethyl]-5-methoxy-α-methyl-2Z-(2-phenylethenyl) benzeneethanamine;
N-[2-(3, 4-dimethoxyphenyl)ethyl]-5-methoxy-α-methyl-2Z-(2-phenylethenyl) benzenethanamine;
N-[2-(3, 4-dimethoxyphenyl) ethyl]-5-methoxy-N, α-dimethyl-2E-(2-phenylethenyl) benzenepropanamine;
N-[2-(3 , 4-dimethoxyphenyl)ethyl]-5-methoxy-N, α-dimethyl-2Z-(2-phenylethenyl) benzenepropanamine;
N-[2-(3, 4-dimethoxyphenyl)ethyl]-5-methoxy-N-methyl-2E-(2-phenylethenyl) benzenepropanamine;
N-[2- (3, 4-dimethoxyphenyl)ethyl]-5-methoxy-α-methyl-2E-(2-phenylethenyl) benzeneethanamine;
N-[2-(3 , 4-dichlorophenyl) ethyl]-5-methoxy-α-methyl-2E-(2-phenylethenyl) benzeneethanamine;
N-[2-(3, 5-dimethoxyphenyl) ethyl]-5-methoxy-α-methyl-2E-(2-phenylethenyl) benzeneethanamine;
N-[3-(3, 4-dimethoxyphenyl) propyl]-5-methoxy-α-methyl-2E-(2-phenylethenyl) benzeneethanamine; or
N-[2-[2E-[2-(4-fluorophenyl)ethenyl]-5-methoxyphenyl]-l-methylethyl]-l-naphthaleneethanamine.

**6.** A pharmaceutical composition comprising a stilbene derivative of formula I as defined in any one of claims 1 to 5 in combination with a pharmaceutically acceptable diluent or carrier.

**7.** A stilbene derivative per se of formula I as defined in any one of claims 1 to 6, provided that: when m = 2, both groups Y are $CH_3O$, $Ar^1$ is p-methoxyphenyl, $R^1$ = methyl, q = 0, n = 0, $R^3$ and $R^4$ are both hydrogen and Alk = methylene;
then $Ar^2$ is other than phenyl, p-methoxyphenyl, 3,4-dimethoxyphenyl and 3,4,5-trimethoxyphenyl.

**8.** A carbonyl compound of the following formula (IV):

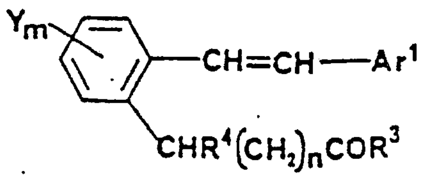

(IV)

wherein
Y, m, $Ar^1$, n, $R^3$ and $R^4$ are as defined in any one of claims 1 to 5.

**9.** A process for the preparation of a stilbene derivative of formula I as defined in any one of claims 1 to 5, which comprises:
a) reducing an acetylene of the following formula (II):

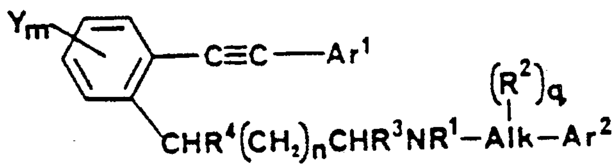

(II)

or
b) reductively alkylating an amine of formula $R^1$-NH-Alk$(R^2)_q$-$Ar^2$ with a compound of formula (IV) as defined in claim 8; or
c) coupling an amine of the following formula (V):

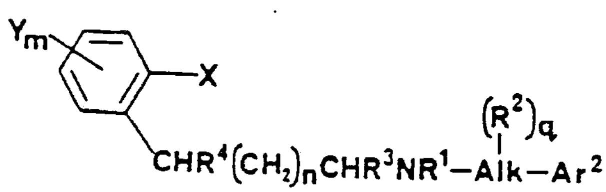

(V)

where X is a halogen with an $Ar^1$-olefin of the formula $Ar^1HC=CH_2$, wherein Y, m, $Ar^1$, $R^1$, $R^2$, $R^3$, $R^4$, n, g, Alk and $Ar^2$ are as defined in any one of claims 1 to 5.

## Revendications

**1.** Dérivé de stilbène de la formule (1) qui suit et ses sels d'addition d'acide acceptables en pharmacie et ses composés d 'ammonium quaternaire à utiliser dans le traitement de l'angine de poitrine ou de l'hypertension chez un mammifère,

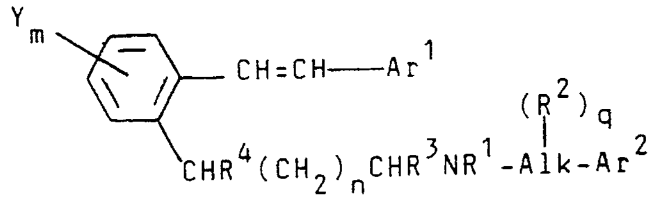

(I)

où

Y est indépendamment alkyle, alcoxy, alkylthio, alkylsulfinyle, alkylsulfonyle, alcanoyloxy, alcanoylamino, amino, monoalkylamino, dialkylamino, hydroxy, halogène ou cyano ou méthylènedioxy ou éthylènedioxy à des carbones adjacents du noyau ;

m est 0, 1 , 2 ou 3 ;

$Ar^1$ est phényle ou phényle indépendamment substitué par un ou plusieurs parmi alkyle, alcoxy, alkylthio, alkylsulfinyle, alkylsulfonyle, amino, alkylamino dialkylamino, carboxamido, halogène, fluoroalkyle ou cyano ;

$R^1$ est hydrogène, alkyle, hydroxyalkyle, cycloalkyle, cycloalkylalkyle ou -Alk-$Ar^2$ ;

$R^2$ est indépendamment hydroxy, alkyle ou phényle ;

Alk est alkylène à chaîne droite de 1 à à 4 carbones ;

q est 0, 1 ou 2 ou 3 si Alk est alkylène de 2 à 4 carbones ;

$Ar^2$ est phényle, phénoxy, thiophénoxy ou naphtyle, lesquels noyaux peuvent être substitués indépendamment par un ou plusieurs parmi alkyle, alcoxy, alkylthio, hydroxy, halogène, fluoroalkyle, nitro, amino ou dialkylamino ou par méthylènedioxy à des carbones adjacents du noyau ;

$R^3$ est hydrogène, alkyle ou alcoxyalkyle ;

n est 0, 1 ou 2 ; et

$R^4$ est hydrogène cu alkyle.

**2.** Dérivé de stilbène de la revendication 1 à utiliser dans le traitement de l'angine de poitrine ou de l'hypertension chez un mammifère, où

Y est alkyle de 1 à 6 carbones ; alcoxy de 1 à 6 carbones ; alkylthio de 1 à 6 carbones ; alkylsulfinyle de 1 à 6 carbones ; alkylsulfonyle de 1 à 6 carbones ; alcanoyloxy de 2 à 6 carbones ; alcanoylamino de 1 à 6 carbones ; amino ; mono-alkylamino de 1 à 6 carbones ; dialkylamino de 2 à 12 carbones ; hydroxy ; fluoro, chloro ou bromo ; cyano ; ou méthylènedioxy ou éthylènedioxy ;

m est 0, 1, 2 ou 3 ;

$Ar^1$ est phényle ou phényle substitué par un ou plusieurs parmi alkyle, alcoxy, alkylthio, alkylsulfinyle, alkylsulfonyle ou alkylamino de 1 à 6 carbones chacun, amino, dialkylamino de 2 à 12 carbones, carboxamido, fluoro, chloro ou bromo, fluoroalkyle de 1 à 6 carbones ou cyano ;

$R^1$ est hydrogène, alkyle de 1 à 6 carbones, hydroxyalkyle de 1 à 6 carbones, cycloalkyle de 3 à 6 carbones, cycloalkylalkyle de 4 à 7 carbones ou -Alk-$Ar^2$ ;

$R^2$ est hydroxy, alkyle de 1 à 4 carbones ou phényle ;

q est 0 ou 1 ;

Alk est méthylène, éthylène, triméthylène ou tétraméthylène ;

$Ar^2$ est phényle, phénoxy, thiophénoxy ou naphtyle, lesquels noyaux peuvent être substitués par un ou plusieurs de alkyle, alcoxy ou alkylthio de 1 à 6 carbones chacun, hydroxy, fluoro, chloro, bromo, fluoroalkyle de 1 à 6 carbones, nitro, amino, dialkylamino de 2 à 12 carbones ou méthylènedioxy ;

$R^3$ est hydrogène, alkyle de 1 à 6 carbones ou alcoxyalkyle de 1 à 6 carbones dans chaque portion alkyle ;

n est 0, 1 ou 2 ; et

$R^4$ est hydrogène ou alkyle de 1 à 6 carbones.

**3.** Dérivé de stilbène de la revendication 1 ou de la revendication 2, à utiliser pour le traitement de l'angine de poitrine ou de l'hypertension chez un mammifère, qui est un cis-stilbène de la formule (I-Z) qui suit :

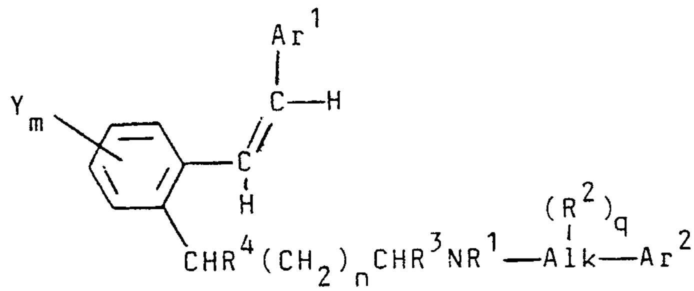

(I-Z)

4. Dérivé de stilbène de la revendication 1 ou de la revendication 2, à utiliser dans le traitement de l'angine de poitrine ou de l'hyerptension chez un mammifère,qui est un trans-stilbène de la formule (I-E) qui suit :

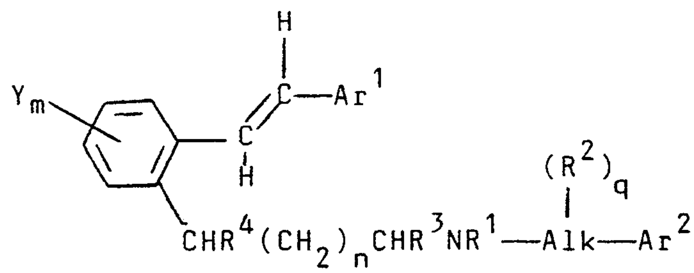

(I-E)

5. Dérivé de stilbène de la revendication 1 à utiliser pour le traitement de l'angine de poitrine ou de l'hypertension chez un mammifère, qui est :
N-[2-(3,4-diméthoxyphényl)éthyl]-5-méthoxy-α-méthyl-2-(2-phényléthényl)benzènepropanamine
N- [2-(2,6-dichlorophényl)éthyl]-5-méthoxy-α - méthyl-2-(2-phényléthényl)benzèneéthanamine ;
N-[2-(3,5-diméthoxyphényl)éthyl]-5-méthoxy-α-méthyl-2-(2-phényléthényl)benzèneéthanamine ;
N- [2-(3 ,4-diméthoxyphényl)éthyl]-5-méthoxy-α-méthyl-2-(2-phényléthényl)benzèneéthanamine ;
N- [2-(3,4-diméthoxyphényl)éthyl]-5-méthoxy-N,α - diméthyl--2(2-phényléthényl)benzènepropanamine ;
N-[2-(3,4-diméthoxyphényl)éthyl]-5-méthoxy-N,α-diméthyl-2-(-2-phényléthényl)benzènepropanamine ;
N- [2-(3,4-diméthoxyphényl)éthyl]-5-méthoxy-N-méthyl-2(2-phényléthényl)benzènepropanamine ;
N-[2-(3,4-diméthoxyphényl)éthyl]-5-méthoxy-α - méthyl-2-(2-phényléthényl)benzèneéthanamine ;
N- [2-(3,4-dichlorophényl)éthyl]-5-méthoxy-α-méthyl-2-(2-phényléthényl)benzèneéthanamine ;
N- [2-(3 ,5-diméthoxyphényl)éthyl]-5-méthoxy-α-méthyl-2-(-2phényléthényl)benzèneéthanamine ;
N- [3-(3 ,4-diméthoxyphényl)propyl]-5-méthoxy-α - méthyl-2-( 2-phényléthényl) benzèneéthanamine;
N-[2- [2-[2-( 4-fluorophényl)éthényl]-5-méthoxyphényl]-1-méthyléthyl]-1-naphtalèneéthanamine ;
N- [2-(3,4-diméthoxyphényl )éthyl]-5-méthoxy-α-méthyl-2E-(2-phényléthényl)benzènepropanamine ;
N-[2-(2,6-dichlorophényl)éthyl-]5-méthoxy-α-méthyl-2Z-(2-phényléthényl)benzèneéthanamine ;
N-[-2(3,s-diméthoxyphényl)éthyl]-5-méthoxy-α-méthyl-2Z-(-2phényléthényl)benzèneéthanamine ;
N- [2-(3,4-diméthoxyphényl)éthyl]-5-méthoxy-α-méthyl-2Z-(2-phényléthényl)benzèneéthanamine ;
N-[2(3,4-diméthoxyphényl)éthyl]5-méthoxy-N,α-diméthyl-2E-(2-phényléthényl)benzènepropanamine ;
N- [2-(3,4-diméthoxyphényl)éthyl]-5-méthoxy-N,α-diméthyl-2Z-(-2-phényéthényl)benzènepropanamine ;
N- [2-(3 ,4-diméthoxyphényl)éthyl]-5-méthoxy-N-méthyl-2E-(2-phényléthényl)benzènepropanamine ;
N-[2-(3,4-diméthoxyphényl)éthyl]-5-méthoxy-α-méthyl-2E(2-phényléthényl)benzèneéthanamine ;
N-[2-(3,4-dichlorophényl)éthyl]-5-méthoxy-α-méthyl-2E-(2-phényléthényl)benzèneéthanamine ;
N-[2-(3,5-diméthoxyphényl)éthyl]-5-méthoxy-α-méthyl-2E-(2-phényléthényl)benzèneéthanamine ;
N-[3-(3,4-diméthoxyphényl)propyl]-5-méthoxy-α-méthyl-2E(2-phényléthényl)benzèneéthanamine ; ou
N-[2-(2E-[2-(4-fluorophényl)éthenyl]-5-méthoxy-phényl]-1-méthyléthyl]-1-naphtalèneéthanamine.

6. Composition pharmaceutique comprenant un dérivé de stilbène de la formule I tel que défini dans l'une

des revendications 1 à 5 en combinaison avec un diluant ou support acceptable en pharmacie.

7. Dérivé de stilbène en lui-même selon la formule 1, tel que défini selon l'une quelconque des revendications 1 à 6, à condition que :
   quand m = 2, les deux groupes Y sont $CH_3O$, $Ar^1$ est p-méthoxyphényle, $R^1$ = méthyle, q = 0, n = 0, $R^3$ et $R^4$ sont tous deux hydrogène et Alk = méthylène;
   $Ar^2$ soit autre que phényle, p-méthoxyphényle, 3,4-diméthoxyphényle et 3,4,5-triméthoxyphényle.

8. Composé de carbonyle de la formule (IV) qui suit :

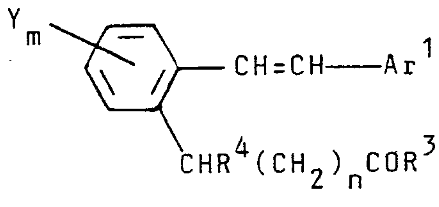

(IV)

où
   Y, m, $AR^1$ , n , $R^3$ et $R^4$ sont tels que définis selon l'une quelconque des revendications 1 à 5.

9. Procédé de préparation d'un dérivé de stilbène de formule I tel que défini selon l'une quelconque des revendications 1 à 5 qui comprend :
   a) la réduction d'un acétylène de la formule suivante (II) :

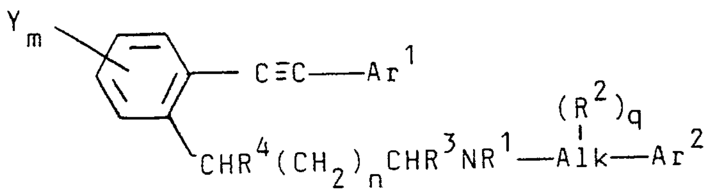

(II)

   ou
   b l'alkylation réductive d'une amine de formule $R^1$- NH-Alk$(R^2)_q$$Ar^2$ avec un composé de formule (IV) tel que défini à la revendication 8 ; ou
   c) le couplage d'une amine de la formule (V) qui suit

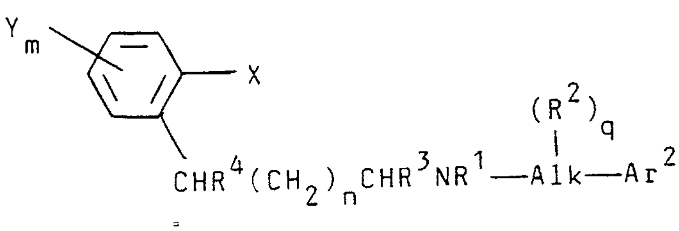

(V)

où X est un halogène avec une Ar $^1$-oléfine de la formule $Ar^1HC=CH_2$, où Y, m, $Ar^1$, $R^1$,$R^2$, $R^3$ $R^4$ n , q , Alk $Ar^1$ $HC=CH_2$, où Y, m, $AR^1$, $R^1$, $R^2$, $R^3$, $R^4$, n, q, Alk et $Ar^2$ sont tels que définis selon l'une quelconque des revendications 1 à 5.

**Ansprüche**

1. Ein Stilbenderivat der nachstehenden Formel (I) und die pharmazeutisch annehmbaren Säureadditionssalze und quaternären Ammoniumverbindungen hievon, zur Anwendung in der Behandlung von Angina Pectoris oder Bluthochdruck in einem Säugetier,

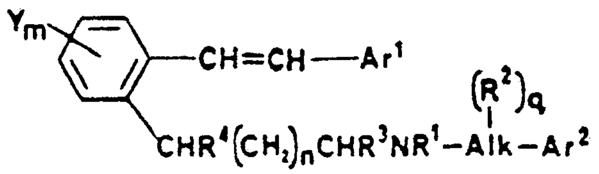

(I),

worin

Y unabhängig voneinander Alkyl, Alkoxy, Alkylthio, Alkylsulfinyl, Alkylsulfonyl, Alkanoyloxy, Alkanoylamino, Amino, Monoalkylamino, Dialkylamino, Hydroxy, Halogen oder Cyan oder Methylendioxy oder Ethylendioxy an benachbarten Ringkohlenstoffatomen bedeutet;

m den Wert 0, 1, 2 oder 3 aufweist;

$Ar^1$ Phenyl bedeutet oder für Phenyl steht, das ein- oder mehrfach, gleich oder verschieden, durch Alkyl, Alkoxy, Alkylthio, Alkylsulfinyl, Alkylsulfonyl, Amino, Alkylamino, Dialkylamino, Carboxamido, Halogen, Fluoralkyl oder Cyan subsituiert ist;

$R^1$ für Wasserstoff, Alkyl, Hydroxyalkyl, Cycloalkyl, Cycloalkylalkyl oder -ALK-$AR^2$ steht;

$R^2$ unabhängig voneinander für Hydroxy, Alkyl oder Phenyl steht;

Alk eine geradkettige Alkylengruppe mit 1 bis 4 Kohlenstoffatomen bedeutet;

q den Wert 0, 1 oder 2 hat oder den Wert 3 aufweist, wenn Alk für Alkylen mit 2 bis 4 Kohlenstoffatomen steht;

$Ar^2$ für Phenyl, Phenoxy, Thiophenoxy oder Naphthyl steht, welche Ringe einfach oder mehrfach, gleich oder verschieden, durch Alkyl, Alkoxy, Alkylthio, Hydroxy, Halogen, Fluoralkyl, Nitro, Amino oder Dialkylamino oder durch Methylendioxy an benachbarten Ringkohlenstoffatomen substituiert sein können;

$R^3$ für Wasserstoff, Alkyl oder Alkoxyalkyl steht;

n den Wert 0, 1 oder 2 aufweist; und

$R^4$ für Wasserstoff oder Alkyl steht.

2. Stilbenderivat nach Anspruch 1 zur Anwendung in der Behandlung von Angina Pectoris oder Bluthochdruck in einem Säugetier, worin

Y für Alkyl mit 1 bis 6 Kohlenstoffatomen; Alkoxy mit 1 bis 6 Kohlenstoffatomen; Alkylthio mit 1 bis 6 Kohlenstoffatomen, Alkylsulfinyl mit 1 bis 6 Kohlenstoffatomen; Alkylsulfonyl mit 1 bis 6 Kohlenstoffatomen; Alkanoyloxy mit 2 bis 6 Kohlenstoffatomen; Alkanoylamino mit 1 bis 6 Kohlenstoffatomen; Amino; Monoalkylamino mit 1 bis 6 Kohlenstoffatomen; Dialkylamino mit 2 bis 12 Kohlenstoffatomen; Hydroxy; Fluor, Chlor oder Brom; Cyan; oder Methylendioxy oder Ethylendioxy steht;

m den Wert 0, 1, 2 oder 3 aufweist;

$Ar^1$ Phenyl bedeutet oder für Phenyl steht, das ein oder mehrfach durch Alkyl, Alkoxy, Alkylthio, Alkylsulfinyl, Alkylsulfonyl oder Alkylamino mit Jeweils 1 bis 6 Kohlenstoffatomen, Amino, Dialkylamino mit 2 bis 12 Kohlenstoffatomen, Carboxamido, Fluor, Chlor oder Brom, Fluoralkyl mit 1 bis 6 Kohlenstoffatomen oder Cyan substituiert ist;

$R^1$ für Wasserstoff, Alkyl mit 1 bis 6 Kohlenstoffatomen, Hydroxyalkyl mit 1 bis 6 Kohlenstoffatomen, Cycloalkyl mit 3 bis 6 Kohlenstoffatomen, Cycloalkylalkyl mit 4 bis 7 Kohlenstoffatomen oder -Alk-$AR^2$ steht;

$R^2$ Hydroxy, Alkyl mit 1 bis 4 Kohlenstoffatomen oder Phenyl bedeutet;

q den Wert 0 oder 1 aufweist;

Alk für Methylen, Ethylen, Trimehtylen oder Tetramethylen steht;

$AR^2$ Phenyl, Phenoxy, Thiophenoxy oder Naphthyl bedeutet, welche Ringe einoder mehrfach durch Alkyl, Alkoxy oder Alkylthio mit Jeweils 1 bis 6 Kohlenstoffatomen, Hydroxy, Fluor, Chlor, Brom, Fluoralkyl mit 1 bis 6 Kohlenstoffatomen, Nitro, Amino, Dialkylamino mit 2 bis 12 Kohlenstoffatomen oder Methylendioxy substituiert sein können;

$R^3$ für Wasserstoff, Alkyl mit 1 bis 6 Kohlenstoffatomen oder Alkoxyalkyl mit 1 bis 6 Kohlenstoffatomen in jedem Alkylteil steht;

n den Wert 0, 1 oder 2 aufweist; und

$R^4$ für Wasserstoff oder Alkyl mit 1 bis 6 Kohlenstoffatomen steht.

3. Stilbenderivat nach Anspruch 1 oder 2, zur Anwendung in der Behandlung von Angina Pectoris oder

Bluthochdruck in einem Säugetier, welches ein cis-Stilben der folgenden Formel (I-Z):

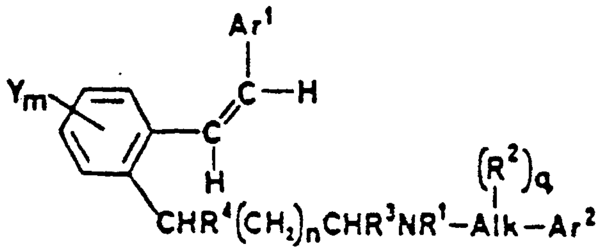

(I-Z)

ist.

4. Stilbenderivat nach Anspruch 1 oder 2, zur Anwendung in der Behandlung von Agina Pectoris oder Bluthochdruck in einem Säugetier, welches ein transStilben der folgenden Formel (I-E):

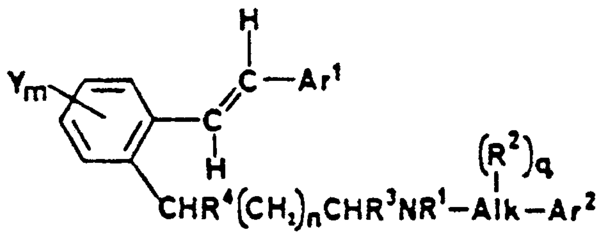

(I-E)

ist.

5. Stilbenderivat nach Anspruch 1, zur Anwendung in der Behandlung von Angina Pectoris oder Bluthochdruck in einem Säugetier, wobei es sich um:
   - N-[2-(3,4-Dimethoxyphenyl)ethyl]-5-methoxy-alpha-methyl-2-(2-phenylethenyl)phenylpropanamin;
   - N-[2-(2,6-Dichlorphenyl)ethyl]-5-methoxy-alpha-methyl-2-(2-phenylethenyl)-Phenylethanamin;
   - N-[2-(3,5-Dimethoxyphenyl)ethyl]-5-methoxy-alpha-methyl-2-(2-phenylethenyl)phenylethanamin;
   - N-[2-(3,4-Dimethoxyphenyl)ethyl]-5-methoxy-alpha-methyl-2-(2-phenylethenyl)phenylethanamin;
   - N-[2-(3,4-Dimethoxyphenyl)ethyl]-5-methoxy-N,alpha-dimethyl-2-(2-phenyl-ethenyl)-phenylpropanamin;
   - N-[2-(3,4-Dimethoxyphenyl)ethyl]-5-methoxy-N,alpha-dimethyl-2-(2-phenyl-ethenyl)-phenylpropanamin;
   - N-[2-(3,4-Dimethoxyphenyl)ethyl]-5-methoxy-N-methyl-2-(2-phenylethenyl)-phenylpropanamin;
   - N-[2-(3,4-Dimethoxyphenyl)ethyl]-5-methoxy-alpha-methyl-2-(2-phenylethenyl)phenylethanamin;
   - N-[2-(3,4-Dichlorophenyl)ethyl]-5-methoxy-alpha-methyl-2-(2-phenylethenyl)-phenylethanamin;
   - N-[2-(3,5-Dimethoxyphenyl)ethyl]-5-methoxy-alpha-methyl-2-(2-phenylethenyl)phenylethanamin;
   - N-[3-(3,4-Dimethoxyphenyl)propyl]-5-methoxy-alpha-methyl-2-(2-phenylethenyl)phenylethanamin;
   - N-[2-[2-[2-(4-Fluorphenyl)ethenyl]-5-methoxyphenyl]-1-methylethyl]-1-naphthylethanamin;
   - N-[2-(3,4-Dimethoxyphenyl)ethyl]-5-methoxy-alpha-methyl-2E-(2-phenylethnyl)phenylpropanamin;
   - N-[2-(2,6-Dichlorphenyl)ethyl]-5-methoxy-alpha-methyl-2Z-(2-phenylethenyl)phenylethanamin;
   - N-[2-(3,5-Dimethoxyphenyl)ethyl]-5-methoxy-alpha-methyl-2Z-(2-phenylethnyl)phenylethanamin;
   - N-[2-(3,4-Dimethoxyphenyl)ethyl]-5-methoxy-alpha-methyl-2Z-(2-phenylethenyl)phenylethanamin;
   - N-[2-(3,4-dimethoxyphenyl)ethyl]-5-methoxy-N,alpha-dimethyl-2E-(2-phenylethenyl)-phenylpropanamin;
   - N-[2-(3,4-Dimethoxyphenyl)ethyl]-5-methoxy-N,alpha-dimethyl-2Z-(2-phenylethenyl)-phenylpropanamin;
   - N-[2-(3,4-Dimethoxyphenyl)ethyl]-5-methoxy-N-methyl-2E(2-phenylethenyl)phenylpropanamin;
   - N-[2-(3,4-Dimethoxyphenyl)ethyl]-5-methoxy-alpha-methyl-2E-(2-phenylethenyl)phenylethanamin;
   - N-[2-(3,4-Dichlorphenyl)ethyl]-5-methoxy-alpha-methyl-2E-(2-phenylethenyl)phenylethanamin;
   - N-[2-(3,5-Dimethoxyphenyl)ethyl]-5-methoxy-alpha-methyl-2E-(2-phenylethenyl)phenylethanamin;
   - N-[3-(3,4-Dimethoxyphenyl)propyl]-5-methoxy-alpha-methyl-2E-(2-phenylethenyl)-phenylethanamin; oder

- N-[2-[2E-[2-(4-Fluorphenyl)ethenyl]-5-methoxyphenyl]-1-methylethyl]-1-naphthylethanamin handelt.

6. Pharmazeutische Zusammensetzung, umfassend ein Stilbenderivat der Formel I, wie in einem der Ansprüche 1 bis 5 definiert, in Kombination mit einem pharmazeutisch annehmbaren Verdünnungsmittel oder Träger.

7. Stilbenderivat als solches der Formel I, wie in einem der Ansprüche 1 bis 6 definiert, mit der Maßgabe, daß dann, wenn m den Wert 2 hat, beide Gruppen Y für $CH_3O$ stehen, $Ar^1$ p-Methoxyphenyl bedeutet, $R^1$ Methyl darstellt, q den Wert O hat, n den Wert O hat, $R^3$ und $R^4$ beide für Wasserstoff stehen und Alk für Methylen steht,
$Ar^2$ eine andere Bedeutung als Phenyl, p-Methoxyphenyl, 3,4-Dimethoxyphenyl oder 3,4,5-Trimethoxyphenyl aufweist.

8. Carbonylverbindung der folgenden Formel (IV):

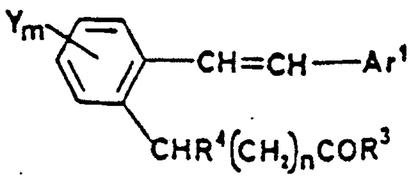

(IV),

worin Y, m, $Ar^1$, n, $R^3$ und $R^4$ wie in einem der Ansprüche 1 bis 5 definiert sind.

9. Verfahren zur Herstellung eines Stilbenderivats der Formel I, wie in einem der Ansprüche 1 bis 5 definiert, welches Verfahren umfaßt:
a) Reduzieren eines Acetylens der folgenden Formel (II):

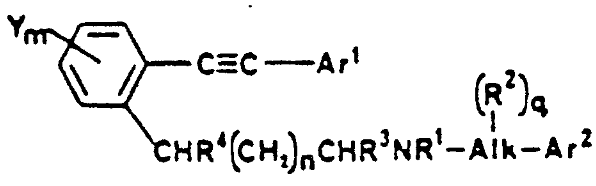

(II);

oder
b) reduktives Alkylieren eines Amins der Formel $R^1$ -NH-Alk$(R^2)_q$-$AR^2$ mit einer Verbindung der Formel (IV), wie in Anspruch 8 definiert; oder
c) Kuppeln eines Amins der folgenden Formel (V):

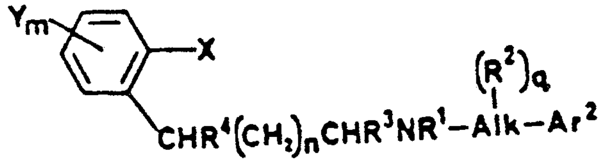

(V),

worin X ein Halogen bedeutet, mit einem $Ar^1$-Olefin der Formel $Ar^1HC=CH_2$, worin Y, m, $Ar^1$, $R^1$, $R^2$, $R^3$, $R^4$, n, q, Alk und $Ar^2$ wie in einem der Ansprüche 1 bis 5 definiert sind.